# EUROPEAN PATENT APPLICATION

(11) **EP 4 015 002 A1**
(43) Date of publication of application: **22.06.2022**
(21) Application number: 20853069.1
(22) Date of filing: 13.08.2020
(51) Int. Cl.: A61K 47/38, A61K 31/496

(54) **ORAL PHARMACEUTICAL COMPOSITION CONTAINING HETEROCYCLIC COMPOUND**

(30) Priority: 13.08.2019 WO PCT/JP2019/031895
(71) Applicant: Otsuka Pharmaceutical Co., Ltd., Tokyo 101-8535 (JP)
(72) Inventor: YOSHIMURA, Motoyasu, Osaka-shi, Osaka 540-0021 (JP); FUJII, Takuya, Osaka-shi, Osaka 540-0021 (JP); KAMADA, Naoki, Osaka-shi, Osaka 540-0021 (JP); TOGASHI, Ryohei, Osaka-shi, Osaka 540-0021 (JP); AONO, Ryuta, Osaka-shi, Osaka 540-0021 (JP); WANG, Xinyu, Osaka-shi, Osaka 540-0021 (JP)
(74) Representative: Hoffmann Eitle
(86) International application number: PCT/JP2020/030777
(87) International publication number: WO 2021/029430

(57) **Abstract**

Provided is a means that is capable of preventing initial excessive release of an active ingredient and that allows for sustained release of the active ingredient in a pharmaceutically active amount over a long period of time.

## Description

### Technical Field

The present disclosure relates to an oral pharmaceutical composition containing a salt of 7-[4-(4-benzo[b]thiophen-4-yl-piperazin-1-yl)butoxy]-1H-quinolin-2-one (more preferably a controlled release oral pharmaceutical composition), and the like. The contents of all of the documents mentioned in the present specification are incorporated herein by reference.

### Background Art

7-[4-(4-Benzo[b]thiophen-4-yl-piperazin-1-yl)butoxy]-1H-quinolin-2-one (hereinafter also referred to as compound (I) or brexpiprazole), or a salt thereof, has a dopamine D₂ receptor partial agonistic action, a serotonin 5-HT_{2A} receptor antagonistic action, and an adrenergic α₁ receptor antagonistic action. In addition to those actions, compound (I) or a salt thereof has a serotonin uptake inhibitory action (or a serotonin reuptake inhibitory action), and is known to have a broad therapeutic spectrum for central nervous system (CNS) diseases (particularly schizophrenia) (Patent Literature (PTL) 1).

### Citation List

### Patent Literature

PTL 1: JP2006-316052A

### Summary of Invention

### Technical Problem

In the treatment of CNS diseases, such as schizophrenia, it is generally important for the drug to be present in plasma at a therapeutically effective concentration over a long period of time. Therefore, a pharmaceutical composition for oral administration that can be administered at low frequency is useful in that it increases patient compliance, and reduces the recurrence rate during treatment. To obtain a pharmaceutical composition for oral administration that can be administered at low frequency, producing a composition containing a high dose of an active ingredient can be considered. However, in order to maintain a blood concentration effective for treatment, the active ingredient is required to be continuously released from the composition at an appropriate rate while preventing excessive release after administration due to factors concerning the living body.

In the treatment of schizophrenia using compound (I) or a salt thereof, once-daily oral administration is recommended at present. However, once-daily oral administration places an undue burden on many patients who require long-term administration. Therefore, there has been a demand for an orally administrable pharmaceutical composition that is suitable for less frequent administration than once-daily administration.

### Solution to Problem

The present inventors conducted extensive research to provide an orally administrable pharmaceutical composition that is suitable for less frequent administration than once-daily administration. As a result, the inventors found that a combination of a fumaric acid salt of compound (I) and a cellulose-based water-soluble polymer may possibly provide an oral solid pharmaceutical composition that exhibits an excellent supersaturated dissolution concentration profile.

The present disclosure includes, for example, the subjects described in the following Items.

### Item 1.

A controlled release oral solid pharmaceutical composition comprising a fumaric acid salt of 7-[4-(4-benzo[b]thiophen-4-yl-piperazin-1-yl)butoxy]-1H-quinolin-2-one as an active ingredient, and further comprising a cellulose-based water-soluble polymer.

### Item 2.

The composition according to Item 1, wherein the cellulose-based water-soluble polymer is at least one member selected from the group consisting of hydroxypropyl methylcellulose, hydroxypropyl cellulose, and methyl cellulose. Item 3.

The composition according to Item 1 or 2, which is an osmotic pump composition.

### Item 4.

The composition according to Item 1 or 2, which is a hydrogel sustained release formulation.

### Item 5.

The composition according to Item 4, comprising an enteric coating.

### Item 6.

The composition according to any one of Items 1 to 5, wherein the fumaric acid salt of 7-[4-(4-benzo[b]thiophen-4-yl-piperazin-1-yl)butoxy]-1H-quinolin-2-one is present in an amount of 5 mg to 60 mg in terms of the weight of the free base.

### Item 7.

The composition according to any one of Items 1 to 6, wherein the blood concentration of 7-[4-(4-benzo[b]thiophen-4-ylpiperazin-1-yl)butoxy]-1H-quinolin-2-one in a steady state when orally administered to a human is maintained in the range of 15 ng/mL to 400 ng/mL for 1 week.

### Item 8.

The composition according to any one of Items 1 to 7, which is for use in administering the fumaric acid salt of 7-[4-(4-benzo[b]thiophen-4-yl-piperazin-1-yl)butoxy]-1H-quinolin-2-one once a week at a dose of 5 mg to 60 mg in terms of the weight of the free base.

### Item 9

The composition according to any one of Items 1 to 8, which is for use in preventing or treating a central nervous system (CNS) disease.

### Item 10.

The composition according to Item 9, wherein the composition is for preventing or treating a CNS disease selected from the group consisting of schizophrenia; treatment-resistant, refractory, or chronic schizophrenia; emotional disturbance; psychotic disorder; mood disorder; bipolar disorder; depression, endogenous depression; major depression; melancholic and treatment-resistant depression; dysthymic disorder; cyclothymic disorder; anxiety disorder; somatoform disorder; factitious disorder; dissociative disorder; sexual disorder; eating disorder; sleep disorder; adjustment disorder; substance-related disorder; anhedonia; delirium; cognitive impairment; cognitive impairment associated with neurodegenerative disease; cognitive impairment caused by neurodegenerative disease; cognitive impairment of schizophrenia; cognitive impairment caused by treatment-resistant, refractory, or chronic schizophrenia; vomiting; motion sickness; obesity; migraine; pain; mental retardation; autism disorder; Tourette's disorder; tic disorder; attention deficit hyperactivity disorder; conduct disorder; Down syndrome; impulsive symptoms associated with dementia; and borderline personality disorder.

### Item A-1.

The composition according to any one of Items 1 to 10 comprising a salt of 7-[4-[4-(4-benzo[b]thiophen-4-yl-piperazin-1-yl)butoxy]-1H-quinolin-2-one as an active ingredient, wherein the composition releases the active ingredient in a sustained manner over a period of 5 to 30 hours.

### Item A-2.

The composition according to Item 3, which is a composition comprising a core formulation comprising a drug layer and a push layer, wherein
the drug layer comprises
   5 to 200 mg of a fumaric acid salt of 7-[4-(4-benzo[b]thiophen-4-yl-piperazin-1-yl)butoxy]-1H-quinolin-2-one in terms of the weight of the free base,
   5 to 94 mass% of a hydrophilic polymer, based on the weight of the drug layer,
   5 to 40 mass% of a cellulose-based water-soluble polymer, based on the weight of the drug layer,
   1 to 50 mass% of an osmagent, based on the weight of the drug layer,
   0.1 to 5 mass% of a lubricant, based on the weight of the drug layer, and
   0.1 to 5 mass% of a fluidizer, based on the weight of the drug layer,
the push layer comprises
   50 to 90 mass% of a highly swellable polymer, based on the weight of the push layer,
   5 to 50 mass% of an osmagent, based on the weight of the push layer,
   0.1 to 5 mass% of a lubricant, based on the weight of the push layer, and
   0.1 to 2 mass% of a pigment, based on the weight of the push layer,
the core formulation comprises 5 to 25 parts by mass of a semipermeable membrane and 1 to 15 parts by mass of a water-soluble polymer membrane, based on 100 parts by mass of the core formulation,
the semi-permeable membrane comprises 70 to 100 mass% of a cellulose-based polymer and 0.01 to 30 mass% of a water-soluble flux-regulating agent, based on the weight of the semipermeable membrane, and
the composition optionally comprising a color coating layer.

### Item A-3.

The composition according to Item 4, comprising:
5 to 200 mg of a fumaric acid salt of 7-[4-(4-benzo[b]thiophen-4-yl-piperazin-1-yl)butoxy]-1H-quinolin-2-one in terms of the weight of the free base,
30 to 90 mass% of a controlled release base material, based on the weight of the core tablet,
1 to 50 mass% of a hydration accelerator, based on the weight of the core tablet,
0.1 to 5% by weight of a lubricant, based on the weight of the core tablet, and
1 to 40 parts by weight of an enteric coating, based on 100 parts by mass of the core formulation.

### Advantageous Effects of Invention

According to the present disclosure, there can be provided a means that prevents initial excessive release of an active ingredient (i.e., a salt of compound (I)) from a pharmaceutical composition, even when the composition is administered in a high dose; and that allows for sustained release of a therapeutically effective amount of the active ingredient over a long period of time. This can maintain a therapeutically effective blood concentration of the active ingredient for a long period of time. Thus, according to the present disclosure, a disease (e.g., schizophrenia) responsive to a salt of compound (I) can be treated by less frequent administration than conventional methods, and the present disclosure is thus effective in improving medication compliance in patients.

Examples of the disease that is responsive to compound (I) or a salt thereof include various CNS diseases such as schizophrenia; treatment-resistant, refractory, or chronic schizophrenia; emotional disturbance; psychotic disorder; mood disorder; bipolar disorder (e.g., bipolar I disorder and bipolar II disorder); depression; endogenous depression; major depression; melancholic and treatment-resistant depression; dysthymic disorder; cyclothymic disorder; anxiety disorder (e.g., panic attack, panic disorder, agoraphobia; social phobia; obsessive-compulsive disorder; post-traumatic stress disorder; generalized anxiety disorder; and acute stress disorder); somatoform disorder (e.g., hysteria, somatization disorder; conversion disorder; pain disorder; and hypochondria); factitious disorder, dissociative disorder; sexual disorder (e.g., sexual dysfunction, libido disorder, sexual arousal disorder, and erectile dysfunction); eating disorder (e.g., anorexia nervosa and bulimia nervosa); sleep disorder; adjustment disorder; substance-related disorder (e.g., alcohol abuse, alcohol intoxication and drug addiction, amphetamine addiction, and narcotism); anhedonia (e.g., loss of pleasure, anhedonia, iatrogenic anhedonia, anhedonia of a psychic or mental cause, anhedonia associated with depression, anhedonia associated with schizophrenia); delirium; cognitive impairment; cognitive impairment associated with Alzheimer's disease, Parkinson's disease, and other neurodegenerative diseases; cognitive impairment caused by Alzheimer's disease, Parkinson's disease, and other neurodegenerative diseases; cognitive impairment of schizophrenia; cognitive impairment caused by treatment-resistant, refractory, or chronic schizophrenia; vomiting; motion sickness; obesity; migraine; pain; mental retardation; autistic disorder (autism); Tourette's syndrome; tic disorder; attention deficit hyperactivity disorder; conduct disorder; Down syndrome; impulsive symptoms associated with dementia (e.g., agitation associated with Alzheimer's disease); and borderline personality disorder.

### Brief Description of Drawings

Fig. 1a shows the results of a dissolution test of oral pharmaceutical compositions (osmotic pump formulations) containing a fumaric acid salt of compound (I) or compound (I).
Fig. 1b shows the results of a dissolution test of oral pharmaceutical compositions (osmotic pump formulations) containing a fumaric acid salt of compound (I) or compound (I).
Fig. 2a shows the results of a dissolution test of oral pharmaceutical compositions (osmotic pump formulations) containing a fumaric acid salt of compound (I).
Fig. 2b shows the results of a dissolution test of oral pharmaceutical compositions (osmotic pump formulations) containing a fumaric acid salt of compound (I).
Fig. 3 shows the results of a dissolution test of oral pharmaceutical compositions (hydrogel sustained release formulations) containing a fumaric acid salt of compound (I).
Fig. 4a shows the infrared absorption spectrum of a fumaric acid salt of compound (I).
Fig. 4b shows the powder X-ray diffraction pattern of a fumaric acid salt of compound (I).
Fig. 5 shows an example of an osmotic pump composition, which is one embodiment of the controlled release oral pharmaceutical composition.

### Description of Embodiments

The present disclosure preferably includes an oral pharmaceutical composition, a method for producing the oral pharmaceutical composition, and the like. However, the disclosure is not limited thereto, and includes everything that is disclosed in the present invention and that can be recognized by one skilled in the art.

The oral pharmaceutical composition according to the present disclosure includes salts of compound (I). The oral pharmaceutical composition may be referred to as the "oral pharmaceutical composition according to the present disclosure." Compound (I) refers to a compound represented by the following formula (I). Compound (I) or a salt thereof can be produced by the method disclosed in JP2006-316052A (the entirety of which is incorporated herein by reference), or a method similar thereto.

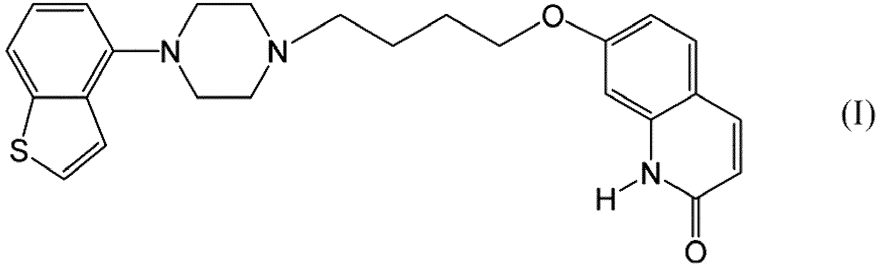

The salt of compound (I) is not particularly limited, insofar as it is a pharmaceutically acceptable salt. Examples include various metal salts, inorganic base salts, organic base salts, inorganic acid salts, organic acid salts, and the like. Examples of metal salts include alkali metal salts (e.g., sodium salts and potassium salts), alkaline earth metal salts (e.g., calcium salts and magnesium salts), and the like. Examples of inorganic base salts include ammonium salts and salts of alkali metal carbonates (e.g., lithium carbonate, potassium carbonate, sodium carbonate, and cesium carbonate), alkali metal hydrogen carbonates (e.g., lithium hydrogen carbonate, sodium hydrogen carbonate, and potassium hydrogen carbonate), alkali metal hydroxides (e.g., lithium hydroxide, sodium hydroxide, potassium hydroxide, and cesium hydroxide), and like inorganic bases. Examples of organic base salts include salts of tri(lower)alkylamines (e.g., trimethylamine, triethylamine, and N-ethyldiisopropylamine), pyridine, quinoline, piperidine, imidazole, picoline, dimethylaminopyridine, dimethylaniline, N-(lower)alkyl-morpholines (e.g., N-methylmorpholine), 1,5-diazabicyclo[4.3.0]nonene-5 (DBN), 1,8-diazabicyclo[5.4.0]undecene-7 (DBU), 1,4-diazabicyclo[2.2.2]octane (DABCO), and like organic bases. Examples of inorganic acid salts include hydrochloric acid salts, hydrobromic acid salts, hydroiodic acid salts, sulfuric acid salts, nitric acid salts, phosphoric acid salts, and the like. Examples of organic acid salts include formic acid salts, acetic acid salts, propionic acid salts, oxalic acid salts, malonic acid salts, succinic acid salts, fumaric acid salts, maleic acid salts, lactic acid salts, malic acid salts, citric acid salts, tartaric acid salts, carbonic acid salts, picric acid salts, methanesulfonic acid salts, ethanesulfonic acid salts, p-toluenesulfonic acid salts, glutamic acid salts, benzoic acid salts, and the like. Among these, hydrochloric acid salts, sulfuric acid salts, fumaric acid salts, phosphoric acid salts, citric acid salts, and tartaric acid salts are preferable. Fumaric acid salts are particularly preferable.

Examples of salts of compound (I) include anhydrides, solvates with a solvent (e.g., hydrate, methanolate, ethanolate, and acetonitrilate), various crystal forms of anhydrides and solvates, and mixtures thereof. Compound (I) or a salt thereof also includes isomers such as geometric isomers, stereoisomers, and optical isomers.

The salt of compound (I) can be a pharmaceutically acceptable co-crystal salt. The term "co-crystal" or "co-crystal salt" as used herein means a crystalline material composed of two or more unique solids at room temperature that are different in physical characteristics (such as structure, melting point, and heat of fusion). Co-crystals and co-crystal salts can be produced by known co-crystallization methods.

The oral pharmaceutical composition according to the present disclosure is suitable for maintaining a constant dissolution concentration of a salt of compound (I), even in the lower part of the gastrointestinal tract. Further, preferably, the oral pharmaceutical composition according to the present disclosure is suitable for releasing a salt of compound (I) at a constant rate over a long period of time. More specifically, the oral pharmaceutical composition is suitable for use as a controlled release oral pharmaceutical composition.

The dissolution rate and sustained release time of the oral pharmaceutical composition can be calculated by measuring the dissolution rate and sustained release time of compound (I) according to the second method (paddle method) of the dissolution test of the Japanese Pharmacopoeia using, as a test solution, a buffer of pH 5.0 or less that achieves sink conditions (specifically, a 0.05 mol/L acetate buffer (pH 4.3, acetic acid, sodium acetate)).

The "dissolution rate" refers to the ratio of the dissolved salt of compound (I) to the total amount of the salt of compound (I) contained in the oral pharmaceutical composition. Therefore, the dissolution rate can be paraphrased as the ratio of dissolved (eluted) compound (I) to the total amount of compound (I) contained in the oral pharmaceutical composition. The "sustained release time" means the time from the start of measurement of the dissolution test until the final dissolution rate has been reached. The "final dissolution rate" means the dissolution rate when a plateau is reached in the dissolution test, and the "final dissolution amount" means the amount of elution when the final dissolution rate is reached in the dissolution test. In the dissolution test, when the dissolution rate at a certain point of time (the reference point of time) is compared with the dissolution rate two hours after the reference point of time, and if the dissolution rate two hours after the reference point of time falls within the range of the dissolution rate at the reference point of time + 1% (preferably, when the dissolution rate at a certain point of time (the reference point of time) is compared with the dissolution rate more than two hours after the reference point of time and if the dissolution rate more than two hours after the reference point of time falls within the range of the dissolution rate at the reference point of time + 1%), a plateau is considered to be reached at the reference point of time that is the shortest in time from the start of the test. However, the dissolution rate at the reference point of time should be more than 20% (in other words, when the dissolution rate is not more than 20%, it is not considered to be a plateau).

The sustained release time is preferably 5 hours or more. The sustained release time is preferably 30 hours or less. More preferably, the sustained release time is 5 to 30 hours. The upper or lower limit of the range of the sustained release time is, for example, 6, 7, 8, 9, 10, 11, 12, 13, 14, 15, 16, 17, 18, 19, 20, 21, 22, 23, 24, 25, 26, 27, 28, or 29 hours. The sustained release time is, for example, even more preferably 10 to 24 hours, and still even more preferably 15 to 24 hours.

The final dissolution amount of the oral pharmaceutical composition according to the present disclosure is preferably 80 mass% or more of the total amount of the salt of compound (I) contained in the oral pharmaceutical composition (typically, the oral pharmaceutical formulation). More preferably, the final dissolution amount is 81, 82, 83, 84, 84, 85, 85, 86, 87, 88, 89, or 90 mass% or more.

Whether a constant dissolution concentration is maintained in the lower part of the gastrointestinal tract can be evaluated by measuring the supersaturated dissolution concentration profile of the salt of compound (I) per dissolution time using as a test liquid a phosphate buffer of around pH 7, which simulates the lower part of the gastrointestinal tract, according to the second method (paddle method) of the dissolution test of the Japanese Pharmacopoeia.

In achieving "sustained release," a rise in initial dissolution of the salt of compound (I) from the composition may be achieved immediately after the start of the dissolution test, or a certain amount of time (for example, 1 to 3 hours) after the start of the measurement. However, it is undesirable for it to take more than 5 hours until the amount of dissolution becomes 5 mass% or more of the final dissolution amount.

The "supersaturated dissolution profile" can be evaluated by quantifying, over time, the concentration of the drug temporarily dissolved at a solubility level higher than that of compound (I) in the paddle method dissolution test using the above phosphate buffer solution of around pH 7 (test liquid that simulates the lower part of the digestive tract). A preferred supersaturated dissolution profile is a profile that reaches a peak of the dissolution rate at a point of time between 4 hours to 18 hours after the start of the dissolution test. The peak is preferably 1 or 1.5 µg/mL or more, more preferably 2, 2.5, or 3 µg/mL or more, and even more preferably 3.5, 4, 4.5, 5, 5.5, or 6 µg/mL or more, in terms of compound (I) (free base). A higher supersaturated concentration contributes more to absorption in the lower part of the gastrointestinal tract, and is thus expected to increase BA (bioavailability) and reduce PTF (Peak Trough Ratio). Such a BA enhancement or PTF reduction makes it easier to fit to the PK (pharmacokinetics) target range.

The mode in which the salt of compound (I) is released from the oral pharmaceutical composition at a uniform rate over a long period of time is not particularly limited, and can be achieved by various techniques known in the field of sustained release formulations. Preferable sustained release approaches are, for example, those using a diffusion-controlled composition, a dissolution-controlled composition, or an osmotic pump controlled release composition. Among these, more preferable sustained release approaches are, for example, an osmotic pump controlled release (especially an osmotic controlled release oral delivery system: OROS) composition, and a hydrogel sustained release composition. Such an oral pharmaceutical composition is preferably an oral solid pharmaceutical composition (particularly a solid formulation) from the viewpoint of being easy to handle.

The oral pharmaceutical composition according to the present disclosure preferably contains a cellulose-based water-soluble polymer. The cellulose-based water-soluble polymer is preferably contained in a drug-containing composition. The cellulose-based water-soluble polymer that can be preferably used is, for example, a cellulose-based water-soluble polymer known in the pharmaceutical field. For example, a cellulose-based water-soluble polymer having a structure in which some of the hydrogen atoms of OH groups of cellulose are replaced with methyl groups and/or hydroxypropyl groups is preferable. Specific examples of preferable cellulose-based water-soluble polymers include hydroxypropylmethyl cellulose, hydroxypropyl cellulose, methyl cellulose, and the like. Such cellulose-based water-soluble polymers can be used singly, or in a combination of two or more.

In particular, when a fumaric acid salt is used as the salt of compound (I), a combination of a fumaric acid salt of compound (I) and a cellulose-based water-soluble polymer can provide an oral solid pharmaceutical composition exhibiting a remarkably excellent supersaturated dissolution profile.

Next, the present disclosure is described below in more detail with reference to an osmotic pump controlled release composition, which is a preferred oral solid pharmaceutical composition. A formulation of the osmotic pump controlled release composition can be referred to as an osmotic pump formulation.

The osmotic pump composition generally has a structure in which a drug and optionally a substance, such as a salt, that generates osmotic pressure (an osmotic agent) are surrounded by a semipermeable membrane; and the semipermeable membrane has pores through which the drug can be released. A fluid (e.g., water) enters through the semipermeable membrane according to the osmotic pressure and dissolves the drug and osmotic agent therein, which increases the osmotic pressure gradient across the semipermeable membrane and causes additional fluid to flow into the semipermeable membrane, thereby further dissolving and releasing the drug. The osmotic pump composition is advantageous in that since the release rate of the drug does not depend on the pH of the environment, even if the composition passes through the gastrointestinal tract and encounters a significantly different pH environment, the composition can sustainably release the drug at a constant rate according to the osmotic pressure over a long period of time.

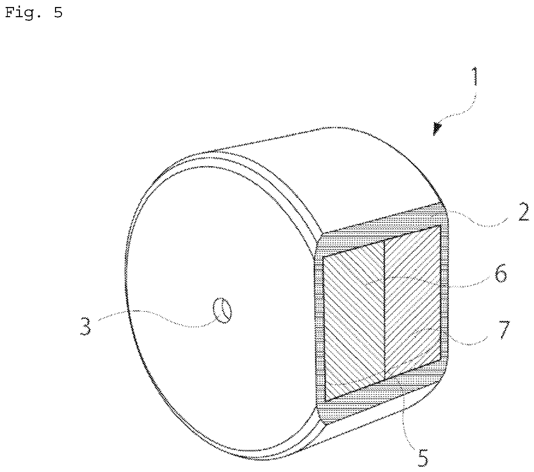

For one embodiment of an oral pharmaceutical composition of the present disclosure, which is an osmotic pump composition, an explanation is provided below with reference to the drawings. In Fig. 5, the osmotic pump composition 1 (hereinafter sometimes referred to simply as formulation 1) includes a wall 2 surrounding an internal compartment 5 in which a composition containing a salt of compound (I) is present. The wall 2 has at least one drug release port 3 that communicates the external environment with the internal compartment. The internal compartment 5 includes a bilayered compressed core comprising a drug layer 6 and a push layer 7. The drug release port 3 is preferably provided in the wall 2 so as to communicate the internal compartment 5 on the drug layer 6 side with the external environment. One or more drug release ports 3 may be provided. For example, the wall may have two or three drug release ports 3.

The wall 2 is a semi-permeable membrane through which water and external liquids permeate, but through which drugs, osmotic agents, and the like do not permeate. The drug layer 6 comprises a salt of compound (I) in the form of a mixture with one or more additives. The push layer 7 does not comprise any salts of compound (I), and comprises an osmotic agent and a highly swelling polymer. The osmotic agent refers to an ingredient that is water-soluble and that raises the concentration of an electrolyte in a pharmaceutical formulation, such as an inorganic salt and a saccharide and/or a sugar alcohol. The highly swellable polymer means a polymer that absorbs a liquid and swells (a polymer having a relatively large molecular weight is preferable). The highly swellable polymer absorbs a liquid and swells, whereby a salt of compound (I) is released through the drug release port 3. The drug layer 6 and the push layer 7 further include additives, such as a hydrophilic polymer, an osmagent, a hydration promoter, a pH regulator, a binder, a fluidizer, an antioxidant, a lubricant, and a pigment.

In an osmotic pump composition, after oral intake, a liquid, such as water, permeates the semi-permeable membrane and is absorbed into the composition. Due to the generated osmotic pressure effect, a salt of compound (I) in the drug layer becomes releasable, and the highly swellable polymer in the push layer swells simultaneously. As the body fluid continues to permeate the internal compartment, a releasable salt of compound (I) can be released through the release port 3. The release of the salt of compound (I) results in further permeation of the body fluid and further swelling of the push layer to achieve a sustained release of the salt of compound (I).

Preferably, the semi-permeable membrane used is highly permeable to external liquids, such as water and biological fluids, but is substantially impermeable to a salt of compound (I), osmagents, highly swellable polymers, etc. Preferably, the semipermeable membrane is substantially non-erodible, and insoluble *in vivo.*

Examples of typical polymers used to form a semipermeable film include semipermeable monopolymers, semipermeable copolymers, and the like. Examples include cellulose-based polymers such as cellulose ester, cellulose ether, and cellulose ester-ether. Specific examples include cellulose acylate, cellulose diacylate, cellulose triacylate, cellulose acetate, cellulose diacetate, cellulose triacetate, mono-, di- and tri-cellulose alkanylate, mono-, di- and tri-alkenylate, mono-, di- and tri-alloylate, and the like. Among them, cellulose acetate is preferable. The semipermeable membrane can be prepared from such a polymer by a known method.

In addition to the above, other examples of the semipermeable polymer for forming a semipermeable membrane of the osmotic pump composition include the following: cellulose acetaldehyde dimethyl acetate; cellulose acetate ethyl carbamate; cellulose acetate methyl carbamate; cellulose dimethylaminoacetate; semipermeable polyurethane; semipermeable sulfonated polystyrene; crosslinked selectively semipermeable polymers formed by co-precipitation from anion and cation, as disclosed in U.S. Patent Nos. 3,173,876, 3,276,586, 3,541,005, 3,541,006, and 3,546,142; semipermeable polymers, as disclosed in U.S. Patent No. 3,133,132; semipermeable polystyrene derivatives; semipermeable poly(sodium styrene sulfonate); semipermeable poly(vinyl benzyltrimethylammonium chloride); and a semipermeable polymer showing a liquid permeability of 10⁻⁵ to 10⁻² (cc ml/cm hr atm) in terms of a hydrostatic pressure difference or an osmotic pressure difference per atmosphere upon permeation through a semipermeable wall.

Such exemplified polymers for use to form the semipermeable membrane may be used singly, or in a combination of two or more.

The semipermeable membrane may contain a flux-regulating agent. The flux-regulating agent means a substance added to assist in regulating the fluid permeability or the fluid volume through the semipermeable membrane. The flux-regulating agent includes a substance that enhances the flux (hereinafter referred to as a flux-enhancing agent) and a substance that decreases the flux (hereinafter referred to as a flux-decreasing agent). The flux-enhancing agent is essentially hydrophilic, while the flux-decreasing agent is essentially hydrophobic.

Examples of the flux-regulating agent include polyhydric alcohols, polyalkylene glycols, polyalkylene diols, polyesters of alkylene glycols, and the like.

Examples of representative flux-enhancing agents include polyethylene glycols having an average molecular weight of 190 to 9000, such as polyethylene glycol 300, 400, 600, 1500, 3000, 3350, 4000, 6000, or 8000; low-molecular-weight glycols, such as polypropylene glycol, polybutylene glycol, and polyamylene glycol; polyalkylenediols, such as poly(1,3-propanediol), poly(1,4-butanediol), and poly(1,6-hexanediol); fatty acids, such as 1,3-butylene glycol, 1,4-pentamethylene glycol, and 1,4-hexamethylene glycol; alkylene triols, such as glycerine, 1,2,3-butanetriol, 1,2,4-hexanetriol, and 1,3,6-hexanetriol; esters, such as ethylene glycol dipropionate, ethylene glycol butyrate, butylene glycol dipropionate, and glycerol acetate esters. Preferred flux-enhancing agents include difunctional block copolymer polyoxyalkylene derivatives of propylene glycol known as Pluronics (BASF), and the like.

Typical flux-decreasing agents include phthalates substituted with alkyl or alkoxy, or with both alkyl and alkoxy groups, such as diethyl phthalate, dimethoxyethyl phthalate, dimethyl phthalate, and [di(2-ethylhexyl)phthalate]; aryl phthalates such as triphenyl phthalate and butyl benzyl phthalate; insoluble salts such as calcium sulfate, barium sulfate, and calcium phosphate; insoluble oxides such as titanium oxide; polymers in the form of, for example, a powder or granules, such as polystyrene, polymethylmethacrylate, polycarbonate, and polysulfone; esters such as citric acid esters esterified with long-chain alkyl groups; inert and water-impermeable fillers; resins compatible with cellulose-based wall forming materials; and the like.

Such exemplified flux-regulating agents for use to form the semipermeable membrane may be used singly, or in a combination of two or more.

The semipermeable membrane may contain other substances, for example, in order to impart flexibility and elongation properties to the semipermeable membrane, to make the semipermeable membrane less brittle, or to give tear strength to the semipermeable membrane. Examples of materials suitably added for such a purpose include plasticizers. Specific examples include phthalate plasticizers such as dibenzyl phthalate, dihexyl phthalate, butyl octyl phthalate, C₆-C₁₁ straight-chain phthalates, diisononyl phthalate, diisodecyl phthalate, and the like. Other examples of plasticizers include non-phthalates such as triacetin, dioctyl azelate, epoxidized tallate, triisoctyl trimellitate, triisononyl trimellitate, sucrose acetate isobutyrate, and epoxidized soybean oil. When a plasticizer such as those mentioned above is present in the semipermeable membrane, the amount of plasticizer is about 0.01 to 30 mass% or more, based on the total amount of all components of the semipermeable membrane.

The push layer contains a composition for pushing a salt of compound (I) and is in a layered arrangement in contact with the drug layer, for example, as shown in Fig. 5. As described above, the push layer comprises a highly swellable polymer that absorbs an aqueous liquid or a biological fluid, and swells to extrude a salt of compound (I) through the release port of the formulation. The highly swellable polymer is preferably a swellable hydrophilic polymer that interacts with water or an aqueous biological fluid, and greatly swells or expands; and typically exhibits a 2- to 50-fold volume increase. The highly swellable polymer can be crosslinked or non-crosslinked. In a preferred embodiment, the polymer is preferably at least crosslinked to create an extended polymer network that is too large to exit the formulation. Accordingly, in a preferred embodiment, the swellable composition is retained in the formulation during its effective life span.

Examples of highly swellable polymers include poly(alkylene oxide) having a number average molecular weight of 10000 to 15000000, such as polyethylene oxide, and poly(alkali carboxymethylcellulose) having a number average molecular weight of 500000 to 3500000 (wherein the alkali is sodium, potassium, or lithium). Other examples of highly swellable polymers include polymers that form hydrogels, such as Carbopol (registered trademark), acidic carboxypolymers, acrylic polymers crosslinked with polyallyl sucrose (also known as carboxypolymethylene), and carboxyvinyl polymers having a molecular weight of 250000 to 4000000; Cyanamer (registered trademark) polyacrylamides; crosslinked water-swellable indenemaleic anhydride polymers; Good-rite (registered trademark) polyacrylic acid having a molecular weight of 80000 to 200000; Aqua-Keeps (registered trademark), acrylate polymer polysaccharides composed of condensed glucose units, such as diester crosslinked polygluran; and the like. Polymers that form hydrogels are disclosed in U.S. Patents Nos. 3,865,108, 4,002,173, 4,207,893, etc.

Such exemplified highly swellable polymers can be used singly, or in a combination of two or more.

The osmagent (sometimes referred to as an osmotic solute or an osmotically effective agent) may be present in both of the drug layer and the push layer. The osmagent (osmotic pressure regulator) is not particularly limited, insofar as it exhibits an osmotic activity gradient across the semipermeable membrane. Examples thereof include inorganic salts, inorganic acids, organic salts, organic acids, saccharides, sugar alcohols, and the like. Examples of inorganic salts include sodium chloride, sodium hydrogen carbonate, sodium carbonate, sodium phosphate (trisodium phosphate), potassium phosphate (tripotassium phosphate), sodium hydrogen phosphate (sodium dihydrogen phosphate, disodium hydrogen phosphate), potassium hydrogen phosphate (potassium dihydrogen phosphate, dipotassium hydrogen phosphate), potassium chloride, lithium chloride, magnesium sulfate, magnesium chloride, potassium sulfate, sodium sulfate, sodium hydrogen sulfite, potassium hydrogen sulfite, lithium sulfate, acidic potassium phosphate, and the like. Examples of inorganic acids include acids that form the above-mentioned inorganic salts. Examples of organic salts include sodium fumarate (disodium fumarate), sodium hydrogen tartrate, sodium hydrogen fumarate (monosodium fumarate), potassium hydrogen tartrate, sodium tartrate, sodium potassium tartrate, sodium malate (disodium malate), sodium hydrogen succinate, sodium succinate (disodium succinate), sodium hydrogen maleate, sodium maleate (disodium maleate), sodium hydrogen citrate (sodium dihydrogen citrate, disodium hydrogen citrate), sodium citrate (trisodium citrate), and the like. Examples of organic acids include acids that form the above-mentioned organic salts. Examples of saccharides and sugar alcohols include mannitol, glucose, lactose, fructose, sucrose, sorbitol, xylitol, erythritol, lactose, and the like. These may be anhydrides, or solvates (such as hydrates). These can be used singly, or in a combination of two or more.

The drug layer preferably contains an additive containing an ion in common with the salt of compound (I). For example, when the salt of compound (I) is a fumarate, examples of additives having an ion in common include fumaric acid, monosodium fumarate, disodium fumarate, and the like. In this case, the ion in common is a fumarate ion. A person skilled in the art would understand, according to the type of salt of compound (I) used, what ion is in common with the salt of compound (I), and would be able to use an additive containing the ion in common. Other examples of additives containing an ion in common with the salt of compound (I) that can be used are as described above.

Examples of solvents suitable for use in manufacturing the osmotic pump composition or components thereof include aqueous or inert organic solvents that do not adversely affect substances used in the composition. Examples of such solvents include at least one member selected from the group consisting of aqueous solvents, alcohols, ketones, esters, ethers, aliphatic hydrocarbons, halogenated solvents, cycloaliphatic, aromatic, or heterocyclic solvents, and mixtures thereof.

Examples of representative solvents include acetone, diacetone alcohol, methanol, ethanol, isopropanol, butyl alcohol, methyl acetate, ethyl acetate, isopropyl acetate, n-butyl acetate, methyl isobutyl ketone, methyl propyl ketone, n-hexane, n-heptane, ethylene glycol monoethyl ether, ethylene glycol monoethyl acetate, methylene dichloride, ethylene dichloride, propylene dichloride, carbon tetrachloride, nitroethane, nitropropane, tetrachloroethane, ethyl ether, isopropyl ether, cyclohexane, cyclooctane, benzene, toluene, naphtha, 1,4-dioxane, tetrahydrofuran, diglyme, water, an aqueous solvent containing an inorganic salt, such as sodium chloride or calcium chloride, and mixtures thereof (such as a mixture of acetone and water, a mixture of acetone and methanol, a mixture of acetone and ethyl alcohol, a mixture of methylene dichloride and methanol, and a mixture of ethylene dichloride and methanol); and the like.

The drug layer comprises a therapeutically effective amount of a salt of compound (I) and a carrier. The carrier can comprise a hydrophilic polymer. The hydrophilic polymer can provide, for example, hydrophilic polymer particles in the pharmaceutical composition that contribute to a uniform release rate and a controlled release pattern of the salt of compound (I). Examples of these polymers include poly(alkylene oxide) having a number average molecular weight of 100000 to 750000, such as poly(ethylene oxide), poly(methylene oxide), poly(butylene oxide), and poly(hexylene oxide); and poly(carboxymethyl cellulose) having a number average molecular weight of 40000 to 400000, such as poly(alkali carboxymethylcellulose), poly(sodium carboxymethylcellulose), poly(potassium carboxymethylcellulose), poly(lithium carboxymethylcellulose), and the like. The pharmaceutical composition may contain hydroxypropylalkyl cellulose having a number average molecular weight of 9200 to 125000, such as hydroxypropylethyl cellulose, hydroxypropylmethyl cellulose, hydroxypropylbutyl cellulose, and hydroxypropylpentyl cellulose, to improve release properties of the composition; and poly(vinylpyrrolidone) having a number average molecular weight of 7000 to 75000, to improve flow properties of the composition. Among these polymers, poly(ethylene oxide) having a number average molecular weight of 100000 to 300000 is preferable.

Other carriers that can be present in the drug layer include carbohydrates that exhibit sufficient osmotic activity when used alone or with another osmagent. Such carbohydrates include monosaccharides, disaccharides, and polysaccharides. Representative examples include saccharides such as maltodextrin (i.e., a glucose polymer produced by hydrolysis of corn starch), lactose, glucose, raffinose, sucrose, mannitol, and sorbitol. Preferred maltodextrins are those having a dextrose equivalence (DE) of 20 or less, preferably a DE of about 4 to about 20, and more preferably a DE of 9 to 20. The use of a maltodextrin having a DE of 9 to 12 is preferable. Carbohydrates (preferably maltodextrin) are preferable because they are usable in the drug layer without adding another osmagent, and impart long-term stability to the composition.

The drug layer is, for example, a homogeneous dry composition formed by compression of a carrier and a drug. The drug layer may be formed from particles of a milled drug and an added polymer. Granulation can be carried out using known techniques, such as granulation, spray drying, sieving, lyophilization, crushing, grinding, shredding, and the like. Such granulation can be carried out using devices such as a high-speed stirring granulator, an extrusion granulator, a fluidized bed granulator, and a roller compactor.

The drug layer may contain one or more surfactants, and one or more disintegrating agents. Examples of such surfactants include surfactants having an HLB value of about 10 to 25 (specifically, polyethylene glycol 400 monostearate, polyoxyethylene-4-sorbitan monolaurate, polyoxyethylene-20-sorbitan monooleate, polyoxyethylene-20-sorbitan monopalmitate, polyoxyethylene-20-monolaurate, polyoxyethylene-40-stearate, sodium oleate, and the like). Examples of disintegrants include starches, clays, celluloses, algins, gums, crosslinked starches, celluloses, polymers, and the like. Preferable disintegrants include corn starch, potato starch, croscarmellose, crospovidone, sodium starch glycolate, Veegum HV, methylcellulose, agar, bentonite, carboxymethylcellulose, alginic acid, guar gum, and the like.

The drug layer may further contain light anhydrous silicic acid and the like.

The semipermeable membrane can be formed on the surface of the bilayered compressed core, for example, by pan coating. More specifically, the semipermeable membrane can be formed by spraying the composition for forming a semipermeable membrane over the surface of the compressed bilayered core comprising the drug layer and the push layer tumbling in a rotating pan. Coating techniques other than pan coating can also be used to coat the compression core. For example, the semipermeable membrane may be formed by a technique using an air-suspension procedure. This procedure consists of suspending the compressed core in a current of air and the semipermeable membrane-forming composition, and tumbling it until a semipermeable membrane is formed on the core. The air-suspension procedure is well-suited for independently forming the semipermeable membrane. Such an air-suspension technique is known (e.g., U.S. Patent No. 2,799,241). It is also possible to use a Wurster (registered trademark) air-suspension coater, or an AeromaticR (registered trademark) air-suspension coater.

After coating with the semi-permeable membrane, the semipermeable membrane is dried, for example, in a forced-air furnace, or in a furnace with a controlled temperature and humidity, to thereby remove the solvent. Drying conditions can be suitably selected in consideration of available equipment, ambient conditions, solvents, coating materials, coating thickness, etc.

The oral pharmaceutical composition according to the present disclosure can be produced by utilizing a known formulation technique. For example, the oral pharmaceutical composition can be manufactured by existing wet granulation techniques or dry granulation techniques. More specifically, for example, in the case of wet granulation techniques, an organic solvent, such as a denatured anhydrous alcohol, is used as a granulating solution; and a drug and an additive are mixed in a granulator and continuously granulated using the solvent, to thereby obtain granules. The granulating solution is added until a wet mixture is formed, and the wet mass mixture is passed through a pre-installed screen on an oven tray. The mixture is then dried in a forced-air oven. Another granulation method is an operation of granulating powder components of each layer in a fluidized bed granulator. After the powder components are dry-mixed in a granulator, the granulating liquid is sprayed onto the powder. The coated powder is dried in a granulator. The dry granules obtained by these methods are sized in a granulator with a crushing mechanism. A lubricant, such as magnesium stearate, is then added and mixed into granules using a blender (e.g., a V-type blender, or a transportable blender (tote blender)). The composition thus obtained is layered by pressing using, for example, a Manesty (registered trademark) press or a Korsch LCT press. To produce a two-layered core, the drug-containing layer is first pressed; and then a wet mixture of the push layer, which has been produced by the wet granulation technique in a similar manner, is pressed against the drug-containing layer. The compressed bilayered core is coated with a water-soluble polymer, if necessary, and then coated with a semipermeable membrane material as described above. One or more outlets (apertures) are provided at the end of the drug layer of the composition. If necessary, a water-soluble overcoat may be applied, and the overcoat coating may be colored or transparent.

Further, for example, when a dry granulation technique is used as an alternative method, a premixed composition is formed into a layered product using a roller compression molding machine, and the layered product is crushed into granules and sized using a screened grinder with a crushing mechanism. A lubricant, such as magnesium stearate, was added to the sized granules in the same manner as above, mixed into granules using a blender, and pressed in the same manner as in the production method described above.

The osmotic pump composition is provided with at least one release port. The release port is provided during manufacture of the composition or during drug delivery by the composition in a liquid environment at the time of use. The expression "release port" includes the meaning of, for example, passages, openings, orifices, and lumens (bores). This expression also includes apertures formed by erosion, dissolution, or leaching of a substance or a polymer from the outer wall. Such a substance or polymer includes, for example, erodible poly(glycolic acid) acid or poly(lactic acid) in the semipermeable membrane; gelatinous filaments; water-removable poly(vinyl alcohol); and leachable compounds such as fluid-removable aperture-forming substances selected from the group consisting of inorganic salts, organic salts, oxides, and carbohydrates. The release port can be formed, for example, by leaching at least one substance selected from the group consisting of sorbitol, mannitol, lactose, fructose, maltitol, maltose, dextrin, glucose, mannose, galactose, talose, sodium chloride, potassium chloride, and sodium citrate to provide orifices having a pore size suitable for uniform release of a drug. The release port can have any shape, such as a round, rectangular, square, or elliptical shape, as long as the drug can be released from the composition at a uniform rate. The osmotic pump composition can have one or more release ports provided at regular intervals. The orifice size of the release port is not particularly limited, as long as the release of the drug can be controlled in cooperation with the compression core. Preferably, the orifice size of the release port is 0.1 mm to 3 mm. To form a release port, for example, a technique of drilling through the semipermeable membrane, such as mechanical drilling and laser drilling, can be used. Devices for forming such a release port are known (e.g., U.S. Patent No. 3,916,899 and U.S. Patent No. 4,088,864).

The amount of the salt of compound (I) in the oral pharmaceutical composition is, for example, in the range of less than about 1 mg to about 200 mg, or more, per dosage form, in terms of the weight of the free base of compound (I). For example, the drug layer of the osmotic pump composition described below in the Examples contains compound (I) in an amount of 30 mg (in terms of free base). The osmotic pump composition has a T₉₀ value of about 10 hours or more. The salt of compound (I) begins to be released at a uniform rate within about 3 to 4 hours after administration, and the release at a uniform rate continues over a long period of at least about 12 hours. The drug release thereafter further continues for several hours until the formulation is consumed.

The oral pharmaceutical compositions according to the present disclosure preferably releases the drug at a relatively uniform release rate over a long period of time. When the oral pharmaceutical composition according to the present disclosure is administered, a peak occurs at a time later than the occurrence of a peak plasma concentration in a steady state after administration of conventional pharmaceutical agents (e.g., immediate-release formulations), and the peak is smaller than the peak observed after administration of conventional pharmaceutical agents; accordingly, the composition can provide a therapeutically effective average steady-state blood concentration.

The present disclosure includes a method of treating disease states and conditions that are responsive to treatment with compound (I) by orally administering the oral pharmaceutical composition according to the present disclosure to a patient. This method is practiced with a formulation that is suitable for the release of compound (I) at a uniform release rate over a period of at least about 4 hours, preferably 5 to 30 hours, more preferably 10 to 24 hours, and even more preferably 15 to 24 hours.

For the treatment of schizophrenia, the practice of the foregoing method is preferable for the purpose of orally administering the oral pharmaceutical composition according to the present disclosure to a patient at a frequency of less than once a day. Other disease states and conditions, which may be clinically diagnosed as symptoms of schizophrenia, can be treated with the controlled release oral pharmaceutical composition according to the present disclosure.

Although this is not limitative, the oral pharmaceutical composition according to the present disclosure preferably maintains a blood concentration of compound (I) in a steady state in the range of, for example, 15 to 400 ng/mL, or 50 ng/ml to 300 ng/mL, for 1 week, when orally administered to a human (in particular, a human adult).

Ordinary tablets (non-release controlled formulations) containing compound (I) in the form of 0.5 mg, 1 mg, and 2 mg tablets are already commercially available in many countries, including Japan, the U.S., and Europe. Ordinary tablets have been confirmed to be safe and effective against CNS diseases, such as schizophrenia, in clinical trials; and detailed pharmacokinetic analysis has been performed. In consideration of such information on ordinary tablets, it can be understood that any oral pharmaceutical composition capable of maintaining a blood concentration of compound (I) in the range of, for example, 15 ng/mL to 400 ng/mL, or 50 ng/mL to 300 ng/mL, in a steady state, when administered to a human, can be used to prevent or treat a CNS disease, such as schizophrenia, in the same manner as ordinary tablets that are already commercially available.

Accordingly, the oral pharmaceutical composition according to the present disclosure can be orally administered less frequently than once daily; for example, it can be orally administered once a week. The oral pharmaceutical composition according to the present disclosure may be administered in one tablet at a time, or two or more tablets at a time; for example, it may be administered in two tablets, three tablets, four tablets, or five tablets at a time. Persons skilled in the art would be able to appropriately determine the dose of the oral pharmaceutical composition according to the present disclosure to achieve the above blood concentration based on, for example, pharmacokinetic information on the ordinary tablets; and evaluations based on the single-dose administration protocol and multiple-dose administration protocol for the oral pharmaceutical composition according to the present disclosure. For example, the dosage per dose can be about 5 to 60 mg, about 10 to 60 mg, about 20 to 60 mg, or about 45 to 60 mg, in terms of the weight of the free base of compound (I).

As described above, among the oral pharmaceutical compositions according to the present disclosure, one particularly preferable embodiment is an oral solid pharmaceutical formulation of an osmotic pump controlled release system. Of the oral solid pharmaceutical formulations of an osmotic pump controlled release system, particularly preferable embodiments are described below in more detail. Note that the following description may partially overlap with the above description. The following description does not prevent application of the above description.

The oral solid pharmaceutical formulation of an osmotic pump controlled release system preferably has a structure in which a core formulation formed by laminating a drug layer and a push layer is coated with a semipermeable membrane, and the drug layer comprises a salt of compound (I). One embodiment of the formulation includes the formulation shown in Fig. 5.

The mass ratio of the drug layer to the push layer is, for example, about 20 to 125 parts by mass of the push layer per 100 parts by mass of the drug layer. The upper limit or the lower limit of this range is, for example, 25, 30, 35, 40, 45, 50, 55, 60, 65, 70, 75, 80, 85, 90, 95, 100, 105, 110, 115, or 120 parts by mass. The range may be, for example, 35 to 100 parts by mass, or 40 to 60 parts by mass.

The mass ratio of the drug layer to the push layer can be, for example, in the range of about 0.8 to 5. The upper limit or the lower limit of the mass ratio can be, for example, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, or 4.9. For example, the mass ratio of the drug layer to the push layer can be in the range of about 1 to 4, or about 2 to 3. More specifically, the mass ratio can be 2.0, 2.1, 2.2, 2.3, 2.4, 2.5, 2.6, 2.7, 2.8, 2.9, or 3.0.

The salt of compound (I) is not particularly limited, as long as it is a pharmaceutically acceptable salt. Examples include the above-mentioned various metal salts, inorganic base salts, organic base salts, inorganic acid salts, organic acid salts, and the like. The salt is preferably, for example, a fumaric acid salt, a hydrochloric acid salt, a sulfuric acid salt, or the like, and particularly preferably a fumaric acid salt.

The salt includes solvates of salts, and may be present in the form of a solvate in the pharmaceutical formulation. Examples of solvates include hydrate, methanol solvate, ethanol solvate, and the like. The solvate may be a monosolvate, disolvate, trisolvate, or the like. For example, the solvate may be a monohydrate, a dihydrate, a trihydrate, or the like. A particularly preferable example of such a solvate of a salt is, for example, fumarate monohydrate.

The amount of the salt of compound (I) in the drug layer can be, for example, in the range of about 5 to 200 mg in terms of the weight of the free base. The upper limit or the lower limit of this range is, for example, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, or 195 mg. The amount of the salt of compound (I) may be, for example, in the range of about 5 to 60 mg, about 15 to 150 mg, or about 20 to 100 mg, in terms of the weight of the free base.

The amount of the salt of compound (I) contained in the drug layer can be, for example, about 1 to 35 mass% of the drug layer. The upper limit or the lower limit of the range is, for example, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, or 34 mass%. The range may be, for example, about 2 to 30 mass%, or about 5 to 20 mass%.

In addition to the salt, the drug layer may contain additives, such as an osmagent and a hydrophilic polymer.

The osmagent contained in the drug layer is not particularly limited, as long as it exhibits an osmotic pressure gradient through the semipermeable membrane. Examples include inorganic salts, inorganic acids, organic salts, organic acids, saccharides, sugar alcohols, and the like. Examples of inorganic salts include sodium chloride, sodium hydrogen carbonate, sodium carbonate, sodium phosphate (trisodium phosphate), potassium phosphate (tripotassium phosphate), sodium hydrogen phosphate (sodium dihydrogen phosphate, disodium hydrogen phosphate), potassium hydrogen phosphate (potassium dihydrogen phosphate, dipotassium hydrogen phosphate), potassium chloride, lithium chloride, magnesium sulfate, magnesium chloride, potassium sulfate, sodium sulfate, sodium hydrogen sulfite, potassium bisulfite, lithium sulfate, acidic potassium phosphate, and the like. Examples of inorganic acids include acids that form the above-mentioned inorganic salts. Examples of organic salts include sodium fumarate (disodium fumarate), sodium hydrogen fumarate (monosodium fumarate), sodium hydrogen tartrate, potassium hydrogen tartrate, sodium tartrate, sodium potassium tartrate, sodium malate (disodium malate), sodium hydrogen succinate, sodium succinate (disodium succinate), sodium hydrogen maleate, sodium maleate (disodium maleate), sodium hydrogen citrate (sodium dihydrogen citrate, disodium hydrogen citrate), sodium citrate (trisodium citrate), and the like. Examples of organic acids include acids that form the above-mentioned organic salts. Examples of sugars and sugar alcohols include mannitol, glucose, lactose, fructose, sucrose, sorbitol, xylitol, erythritol, lactose, and the like. These may be anhydrides, or solvates (such as hydrates). These can be used alone, or in a combination of two or more. The osmagent contained in the drug layer preferably contains an ion in common with the salt of compound (I). For example, when the salt is a fumaric acid salt, examples of the osmagent containing an ion in common with the fumaric acid salt include fumaric acid, monosodium fumarate, disodium fumarate, and the like. The osmagent can be used singly, or in a combination of two or more.

The content of the osmagent in the drug layer is, for example, about 5 to 40 mass%, based on the weight of the drug layer. The upper limit or the lower limit of the range is, for example, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, or 49 mass%. For example, the range may be 10 to 38 mass% or 15 to 35 mass%.

Examples of hydrophilic polymers contained in the drug layer include polyethylene oxide, polyethylene glycol, and the like. Preferably, the polyethylene oxide is a low-viscosity polyethylene oxide. More specifically, the polyethylene oxide is preferably a polyethylene oxide having an average molecular weight of about 100000 to 300000, and more preferably a polyoxyethylene oxide having an average molecular weight of about 150000 to 250000, or 180000 to 220000. Preferably, the polyethylene glycol is, for example, one in which about 4000 to 8000, preferably about 4500 to 7500, about 5000 to 7000, or about 5500 to 6500 ethylene oxide units are polymerized on average. In order to inhibit recrystallization of the drug in the formulation, the drug layer may contain, as a hydrophilic polymer, a cellulose-based water-soluble polymer such as methyl cellulose, hydroxypropyl cellulose, hydroxypropylalkyl cellulose (e.g., hydroxypropyl ethylcellulose, hydroxypropyl methylcellulose, hydroxypropyl butylcellulose, and hydroxypropylpentyl cellulose), polyvinyl alcohol, polyvinyl alcohol/polyethylene glycol graft copolymer, polyvinylpyrrolidone, copolyvidone, and the like. The hydroxypropylalkyl cellulose is particularly preferably hydroxypropylmethyl cellulose. The hydroxypropyl methylcellulose preferably has a methoxy group content of, for example, about 16 to 30%, and more preferably about 27 to 30%. Such hydrophilic polymers can be used singly, or in a combination with two or more types.

As described above, the oral pharmaceutical composition according to the present disclosure preferably comprises a drug-containing composition containing a cellulose water-soluble polymer. Accordingly, the drug layer preferably contains a cellulose-based water-soluble polymer as a hydrophilic polymer.

The hydrophilic polymer content of the drug layer is, for example, about 5 to 94 mass% of the drug layer. The upper or lower limit of the range are, for example, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 55, 56, 57, 58, 59, 60, 61, 62, 63, 64, 65, 66, and 67, 68, 69, 70, 71, 72, 73, 74, 75, 76, 77, 78, 79, 80, 81, 82, 83, 84, 84, 85, 86, 87, 88, 89, 90, 91, 92, or 93 mass%. For example, the range may be from 20 to 90 mass%.

When the drug-containing composition particularly contains a cellulose-based water-soluble polymer, the content of the cellulose-based water-soluble polymer in the drug layer is, for example, about 5 to 40 mass%, or about 10 to 30 mass%, or about 10 to 20 mass%, based on the weight of the drug layer.

When the drug layer particularly contains (i) polyethylene oxide and (ii) at least one member selected from the group consisting of hydroxypropyl cellulose, methyl cellulose, hydroxypropylalkyl cellulose, polyvinyl alcohol, a polyvinyl alcohol-polyethylene glycol graft copolymer, polyvinylpyrrolidone, and copolyvidone at the same time as hydrophilic polymers, the content of component (i) can be 10 to 60 mass% and the content of component (ii) can be 5 to 40 mass%, based on the weight of the drug layer.

The drug layer may also contain other additives. Examples of such additives include lubricants, fluidizers, and the like. Examples of preferable lubricants include magnesium stearate. Examples of preferable fluidizers include silicon dioxide (in particular, light anhydrous silicic acid).

The drug layer contains a lubricant (in particular, magnesium stearate), for example, in an amount of about 0.1 to 5 mass% or about 0.2 to 3 mass%, based on the weight of the drug layer. The drug layer can contain a fluidizer (in particular, silicon dioxide) in an amount of, for example, 0.1 to 5 mass% or about 0.1 to 3 mass%, based on the weight of the drug layer.

The push layer also comprises a component for extruding a salt of compound (I). Examples of the component include a highly swellable polymer. Examples of highly swellable polymers include polyalkylene oxide, and more specifically include polyethylene oxide. Preferably, the polyethylene oxide contained in the push layer is a high-viscosity polyethylene oxide; and is more specifically, for example, a polyethylene oxide having an average molecular weight of, for example, about 3000000 to 7000000, about 4000000 to 7000000, or about 4000000 to 6000000.

The amount of the highly swellable polymer in the push layer may vary depending on factors, such as the properties and content of the drug in the drug layer; however, it can be any amount that allows the drug to be eluted from the drug layer at a desired release rate by swelling. For example, the content of the highly swellable polymer is 50 to 90 mass%, 50 to 85 mass%, 50 to 80 mass%, 55 to 75 mass%, or about 60 to 70 mass%, based on the weight of the push layer.

The push layer may also contain other additives. For example, the push layer can contain an osmagent. Examples of osmagents in the push layer include inorganic salts, inorganic acids, organic salts, organic acids, saccharides, sugar alcohols, and the like. Examples of inorganic salts include sodium chloride, sodium hydrogen carbonate, sodium carbonate, sodium phosphate), sodium hydrogen phosphate (sodium dihydrogen phosphate, disodium hydrogen phosphate), potassium hydrogen phosphate (potassium dihydrogen phosphate, dipotassium hydrogen phosphate), potassium phosphate (tripotassium phosphate), potassium chloride, lithium chloride, magnesium sulfate, magnesium chloride, potassium sulfate, sodium sulfate, sodium hydrogen sulfite, potassium hydrogen sulfite, lithium sulfate, acidic potassium phosphate, and the like. Examples of inorganic acids include acids forming the inorganic salts described above. Examples of organic salts include sodium fumarate (disodium fumarate), sodium hydrogen fumarate (monosodium fumarate), sodium hydrogen tartrate, potassium hydrogen tartrate, sodium tartrate, sodium potassium tartrate, sodium malate (disodium malate), sodium hydrogen succinate, sodium succinate (disodium succinate), sodium hydrogen maleate, sodium maleate (disodium maleate), sodium hydrogen citrate (sodium dihydrogen citrate, disodium hydrogen citrate), sodium citrate (trisodium citrate), and the like. Examples of organic acids include acids forming the organic salts described above. Examples of saccharides and sugar alcohols include mannitol, glucose, lactose, fructose, sucrose, sorbitol, xylitol, erythritol, lactose, and the like. These may be anhydrides, or solvates (such as hydrates).

Preferable examples of the osmagent contained in the push layer includes sodium chloride, potassium chloride, sodium hydrogen fumarate, sodium fumarate, sodium hydrogen carbonate, sodium carbonate, sodium hydrogen phosphate, sodium phosphate, potassium hydrogen phosphate, potassium phosphate, sodium sulfate, fructose, sucrose, xylitol, sorbitol, glucose, mannitol, erythritol, and lactose. Sodium hydrogen carbonate is particularly preferable. Such osmagents can be used singly, or in a combination of two or more kinds.

In the push layer, the osmagent is preferably present in an amount of, for example, 5 to 50 mass%, 15 to 50 mass%, 20 to 50 mass%, 25 to 45 mass%, or about 30 to 40 mass%, based on the weight of the push layer.

The push layer may further contain other additives. Examples of such additives include lubricants, fluidizers, pigments, and the like. Preferable examples of lubricants include magnesium stearate. Preferable examples of fluidizers include silicon dioxide (in particular, light anhydrous silicic acid). Preferable examples of pigments include iron oxide.

The push layer can contain a lubricant (in particular, magnesium stearate) in an amount of, for example, 0.1 to 5 mass%, based on the weight of the push layer. The push layer can contain a fluidizer (in particular, silicon dioxide) in an amount of, for example, about 0.1 to 5 mass% or 0.1 to 3 mass%, based on the weight of the push layer. Further, the push layer can contain a pigment (in particular, iron oxide) in an amount of, for example, 0.1 to 2 mass%.

The semipermeable membrane with which the core formulation is coated includes, for example, a cellulose-based polymer, preferably cellulose acetate, as described above. The semipermeable membrane may also contain a flux-regulating agent. As described above, the flux-regulating agent is preferably, for example, polyethylene glycol (in particular, polyethylene glycol having an average molecular weight of about 2000 to 6000, about 3000 to 5000, or about 3000 to 6000).

The semipermeable membrane can contain a cellulose polymer in an amount of, for example, about 70 to 100 mass%, or 75 to 95 mass%, based on the weight of the semipermeable membrane. The semipermeable membrane can contain a flux-regulating agent in an amount of about 0.01 to 30 mass% or about 5 to 25 mass%, based on the weight of the semipermeable membrane.

The coating amount of the semipermeable membrane is preferably an amount that allows high permeability to external fluids, such as water and biological fluids; and that is substantially impermeable to salts of compound (I), osmagents, highly swellable polymers, and the like. The amount of the semipermeable membrane can be, for example, in an amount of about 5 to 25 parts by mass per 100 parts by mass of the core formulation. The upper limit or the lower limit of this range is, for example, 6, 7, 8, 9, 10, 11, 12, 13, 14, 15, 16, 17, 18, 19, 20, 21, 22, 23, or 24 parts by mass. The range can be, for example, about 5 to 25 parts by mass.

A water-soluble polymer coating may be applied between the core formulation and the semipermeable membrane in the oral solid pharmaceutical formulation of an osmotic pump controlled release system. In other words, the formulation may have a structure in which a water-soluble polymer membrane and a semipermeable membrane are laminated (preferably applied for coating) in this order on the core formulation.

The water-soluble polymer membrane may contain, as a water-soluble polymer, those described above as hydrophilic polymers that can be contained in the drug layer. Among such polymers, for example, hydroxypropylmethyl cellulose, polyvinyl alcohol, a polyvinyl alcohol-polyethylene glycol graft copolymer, and polyvinylpyrrolidone are preferable. Such water-soluble polymers can be used singly, or in a combination of two or more. When the water-soluble polymer membrane contains hydroxypropylmethylcellulose, for example, 60 to 100 mass% of the water-soluble polymer film may be hydroxypropylmethylcellulose. When the water-soluble polymer membrane contains polyvinylpyrrolidone, for example, 0 to 40 mass% of the water-soluble polymer film may be polyvinylpyrrolidone.

The water-soluble polymer membrane can be present, for example, in an amount of about 1 to 15 parts by mass, per 100 parts by mass of the core formulation.

The oral solid pharmaceutical formulation of the present disclosure can be used, for example, as bare tablets containing the above-mentioned components and having no color coating layer. For example, from the viewpoint of imparting distinctiveness to the formulation, or long-term storage stability and preventing degradation by light, it is preferable to make tablets coated with a color coating layer. If necessary, the coating layer may additionally contain pharmaceutical additives that are usually used in coating treatment (film formation) of pharmaceutical products for oral administration, such as a coating agent, a plasticizer, a dispersant, and an antifoaming agent. If a color coating layer is provided, the color coating layer is preferably the outermost layer. A known coating agent can be used for the color coating layer. For example, a representative premix additive, such as Opadry, can be used as the coating agent. Examples of preferable coating agents include coating agents comprising hydroxypropyl methylcellulose, polyvinyl alcohol, a polyvinyl alcohol-polyethylene glycol graft copolymer, a polyvinyl alcohol-acrylic acid-methyl methacrylate copolymer, or the like as a base material; and further comprising a coloring agent, a lubricant, a plasticizer, or the like.

A preferred example of the oral solid pharmaceutical formulation of an osmotic pump controlled release system according to the present disclosure is a formulation comprising a core formulation comprising a drug layer and a push layer, wherein
the drug layer comprises
   5 to 200 mg of a salt of compound (I) in terms of the weight of the free base,
   5 to 94 mass% of a hydrophilic polymer (containing 5 to 40 mass of a cellulose-based water-soluble polymer, based on the weight of the drug layer) based on the weight of the drug layer,
   1 to 50 mass% of an osmagent, based on the weight of the drug layer,
   0.1 to 5 mass% of a lubricant, based on the weight of the drug layer, and
   0.1 to 5 mass% of a fluidizer, based on the weight of the drug layer,
the push layer comprises
   50 to 90 mass% of a highly swellable polymer, based on the weight of the push layer,
   5 to 50 mass% of an osmagent, based on the weight of the push layer,
   0.1 to 5 mass% of a lubricant, based on the weight of the push layer, and
   0.1 to 2% by weight of a pigment, based on the weight of the push layer,
the core formulation comprises 5 to 25 parts by mass of a semipermeable membrane and 1 to 15 parts by mass of a water-soluble polymer membrane, based on 100 parts by mass of the core formulation,
the semi-permeable membrane comprises 70 to 100 mass% of a cellulose-based polymer and 0.01 to 30 mass% of a water-soluble flux-regulating agent, based on the weight of the semipermeable membrane, and
the formulation optionally comprising a color coating layer.

As described above, another preferable embodiment of the oral pharmaceutical composition according to the present disclosure can be, for example, a hydrogel sustained release composition. More specifically, the hydrogel sustained release composition (hydrogel sustained release formulation) according to the present disclosure preferably contains a fumaric acid salt of compound (I) as an active ingredient, and further contains a cellulose-based water-soluble polymer. The hydrogel sustained release composition is preferably, for example, a hydrogel matrix tablet. Hydrogel matrix tablets are a known technique in which the release of a drug is controlled by a hydrogel, which is formed by (in the case of an enteric-coated tablet, dissolution of the coating film due to a pH increase after gastric excretion, and then) absorption of water in the gastrointestinal tract.

As the sustained release base (hydrogel-forming base) in the hydrogel matrix tablet, for example, the hydrophilic polymers mentioned above can be used. Specific examples of usable bases include cellulose-based water-soluble polymers, polyalkylene oxide (e.g., polyethylene oxide), polyalkylene glycol (e.g., polyethylene glycol), polyvinyl alcohol, and the like. As the sustained release base material, the above hydrophilic polymers can be used singly, or in a combination of two or more. It is particularly preferable that the sustained release base material contains at least one cellulose-based water-soluble polymer. When a hydrophilic polymer other than cellulose-based water-soluble polymers (for example, polyethylene oxide) is mainly used as the sustained release base, the hydrophilic polymer is preferably combined with at least one cellulose-based water-soluble polymer.

As the cellulose-based water-soluble polymer, for example, a cellulose-based water-soluble polymer known in the field of pharmaceutical science can be preferably used. Preferable examples include cellulose-based water-soluble polymers having a structure in which hydrogen atoms of some of the OH groups of the cellulose are replaced with methyl and/or hydroxypropyl groups. Specific examples include hydroxypropyl methylcellulose, hydroxypropyl cellulose, methylcellulose, and the like. The cellulose-based water-soluble polymers can be used singly, or in a combination of two or more.

The cellulose-based water-soluble polymer can be any cellulose-based water-soluble polymer. For example, a cellulose water-soluble polymer having a viscosity of 2.5 to 35000 mm²/s, as measured in the form of a 2% aqueous solution, can be used. It is particularly preferable to use a cellulose-based water-soluble polymer having a viscosity of 2.5 to 17.5 mm²/s.

When a cellulose-based water-soluble polymer (e.g., hypromellose) is mainly used as a sustained release base, the cellulose-based water-soluble polymer has a viscosity of 80 to 35000 mm²/s, as measured in the form of a 2% aqueous solution. The phrase "mainly" using a hydrophilic polymer herein means that the amount of the hydrophilic polymer is 50 mass% or more, for example, 80 mass% or more, or 90 mass% or more, based on the total mass of the sustained release base.

The core tablet can contain a sustained release base in an amount of, for example, about 30 to 90 mass%, or about 50 to 80 mass%, based on the weight of the core tablet.

The hydrogel matrix tablet can further contain other additives. Examples of such additives include a hydration accelerator, a lubricant, a fluidizer, and the like.

Examples of the hydration accelerator include inorganic salts, inorganic acids, organic salts, organic acids, saccharides, sugar alcohols, and the like. Examples of inorganic salts include sodium chloride, sodium hydrogen carbonate, sodium carbonate, sodium phosphate (trisodium phosphate), potassium phosphate (tripotassium phosphate), sodium hydrogen phosphate (sodium dihydrogen phosphate, disodium hydrogen phosphate), potassium hydrogen phosphate (potassium dihydrogen phosphate, dipotassium hydrogen phosphate), potassium chloride, lithium chloride, magnesium sulfate, magnesium chloride, potassium sulfate, sodium sulfate, sodium hydrogen sulfite, potassium hydrogen sulfite, lithium sulfate, acidic potassium phosphate, and the like. Examples of inorganic acids include acids that form the above-mentioned inorganic salts. Examples of organic salts include sodium fumarate (disodium fumarate), sodium hydrogen fumarate (monosodium fumarate), sodium hydrogen tartrate, potassium hydrogen tartrate, sodium tartrate, sodium potassium tartrate, sodium malate (disodium malate), sodium hydrogen succinate, sodium succinate (disodium succinate), sodium hydrogen maleate, sodium maleate (disodium maleate), sodium hydrogen citrate (sodium dihydrogen citrate, disodium hydrogen citrate), sodium citrate (trisodium citrate), and the like. Examples of organic acids include acids that form the above-mentioned organic salts. Examples of saccharides and sugar alcohols include mannitol, glucose, lactose, fructose, sucrose, sorbitol, xylitol, erythritol, lactose, and the like. These may be anhydrides, or solvates (such as hydrates). These can be used singly, or in a combination of two or more. The hydration accelerator is preferably a compound that contains an ion is in common with a fumaric acid salt of compound (I). Examples include fumaric acid, monosodium fumarate, disodium fumarate, and the like. The hydration accelerator preferably contains a hydration accelerator in an amount of, for example, about 1 to 50 mass%, or about 10 to 30 mass%, based on the weight of the core tablet.

Examples of preferable lubricants include magnesium stearate. The hydrogel matrix tablet can contain a lubricant in an amount of, for example, about 0.1 to 5 mass%, or about 0.2 to 3 mass%, based on the weight of the core tablet. Examples of preferable fluidizers include silicon dioxide (in particular, light anhydrous silicic acid). The hydrogel matrix tablet can contain a fluidizer in an amount of, for example, about 0.1 to 5 mass%, or about 0.1 to 3 mass%, based on the mass of the core tablet.

The hydrogel matrix tablet more preferably comprises an enteric coating. A known enteric coating composition can be used for enteric coating. For example, an enteric coating composition containing an enteric base such as Eudragit, a plasticizer such as triethyl citrate, and a lubricant such as talc, can be preferably used. The hydrogel matrix tablet can preferably contain an enteric coating in an amount of, for example, about 1 to 40 parts by mass, or about 10 to 30 parts by mass, per 100 parts by mass of the core tablet.

A preferred example of the hydrogel sustained release formulation according to the present disclosure is a formulation containing 5 to 200 mg of a fumaric acid salt of compound (I) in terms of the weight of the free base; further containing 30 to 90 mass% of a sustained release base material, based on the weight of the core tablet, 1 to 50 mass% of a hydration accelerator, based on the weight of the core tablet, and 0.1 to 5 mass% of a lubricant, based on the weight of the core tablet; and further comprising an enteric coating in an amount of 1 to 40 parts by mass, based on 100 parts by mass of the core formulation.

In this specification, the term "comprising" includes "consisting essentially of" and "consisting of." Further, the present disclosure includes any and all combinations of the components described herein.

Various characteristics (properties, structures, functions, etc.) described in the above embodiments of the present disclosure may be combined in any manner to specify the subject matter included in the present disclosure. That is, this disclosure includes all of the subject matter comprising any combination of the combinable properties described herein.

### Examples

The present invention is described below more specifically with reference to Examples, Test Examples, etc. However, the present disclosure is not limited to these Examples. The compound (I) means 7-[4-(4-benzo[b]thiophen-4-yl-piperazin-1-yl)butoxy]-1H-quinolin-2-one.

### Preparation of 7-[4-(4-benzo[b]thiophen-4-yl-piperazin-1-yl)-butoxy]-1H-quinolin-2-one fumarate

A suspension of 4.22 kg of fumaric acid in 32.5L of water and 22.16 kg of ethanol was stirred under reflux to dissolve fumaric acid (reflux temperature: about 82°C). The obtained solution was filtered while washing with 11.86 kg of ethanol to obtain a fumaric acid solution. A suspension of 15.0 kg of 7-[4-(4-benzo[b]thiophen-4-yl-piperazin-1-yl)-butoxy]-1H-quinolin-2-one in 25.96 kg of water, 8.32 kg of acetic acid, and 34.0 L of ethanol was stirred under reflux to dissolve 7-[4-(4-benzo[b]thiophen-4-yl-piperazin-1-yl)-butoxy]-1H-quinolin-2-one (reflux temperature: about 83°C). The obtained solution was added to the fumaric acid solution, and then filtered while washing with 11.86 kg of ethanol. The filtrate was stirred under reflux for 15 minutes (reflux temperature: about 82°C), then cooled to 30°C or lower, and separated into a solid and a liquid. The obtained solid was washed with water, dried at 80°C, and then moistened to obtain 16.86 kg of 7-[4-(4-benzo[b]thiophen-4-yl-piperazin-1-yl)-butoxy]-1H-quinolin-2-one fumarate.

### Infrared Absorption Spectrum

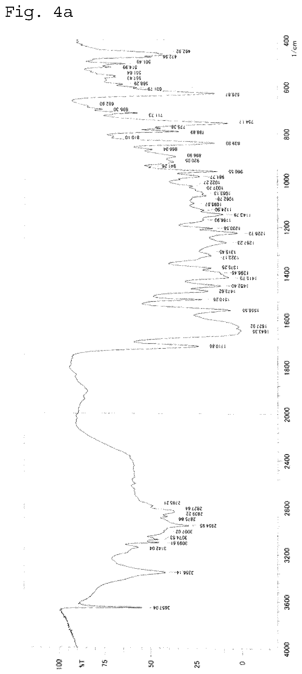

The IR spectrum of the fumaric acid salt prepared by the above method was measured by the KBr tablet method using a Fourier transform infrared spectrophotometer (IRPrestige-21) manufactured by Shimadzu Corporation. As shown in Fig. 4a, the IR spectrum showed absorption at wavenumbers of 3657 cm⁻¹, 1711 cm⁻¹, 1643 cm⁻¹, 1416 cm⁻¹, 1227 cm⁻¹, and 839 cm⁻¹.

### Powder X-Ray Diffraction

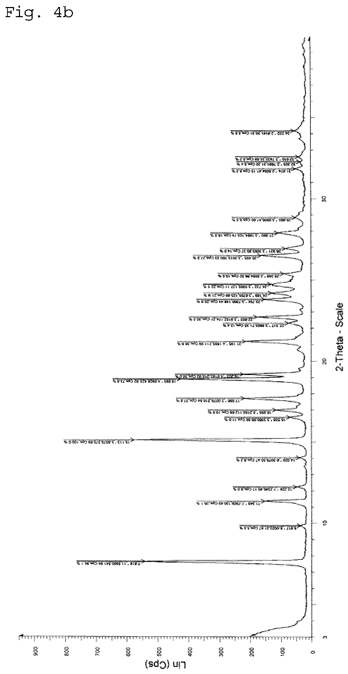

The powder X-ray diffraction of the fumaric acid salt prepared by the above method was measured using an X-ray diffractometer (D8 Advance) manufactured by Bruker AXS. Fig. 4b shows the powder X-ray diffraction pattern of the fumaric acid salt. As shown in Fig. 4b, diffraction peaks were observed at 2θ = 7.6°, 15.1°, 17.7°, 18.9°, and 19.2°. Other peaks were observed at 2θ = 9.8°, 11.3°, 12.2°, 14.0°, 16.5°, 17.0°, 21.2°, 22.3°, 22.7°, 23.8°, 24.2°, 24.7°, 25.4°, 26.5°, 26.9°, 27.9°, 28.9°, 31.9°, 32.3°, 32.6°, and 34.2°.

### Measurement of Water Content

The water content of the prepared fumaric acid salt was measured. Specifically, the water content was measured by the Karl Fischer method (coulometric titration method) using a water content measuring device (Titrando 852) manufactured by Metrohm. The results confirmed that the water content of the fumaric acid salt was 3.01 wt.%.

### Preparation of Drug Sustained Release Formulation and Evaluation 1

An osmotic pump controlled release composition (formulation) comprising a bilayered compressed core comprising a drug layer for providing sustained release of a salt of compound (I) and a push layer was produced according to a known general production process. More specifically, a drug layer composition containing a salt of compound (I) and other inert agents, and a push layer composition containing an osmotic agent and a highly viscous polymer were separately produced; and each composition was then compressed into a bilayered tablet core using known core compression techniques.

Subsequently, the bilayered compressed core was coated with a composition comprising a water-soluble polymer. As a composition of a water-soluble polymer, hypromellose (hydroxypropyl methylcellulose; TC-5, produced by Shin-Etsu Chemical Co., Ltd.) and povidone (Polyvinylpyrrolidone; Kollidon K30, BASF) (70:30 (W/W%)) were dissolved in water to 8% in terms of solids content to prepare a coating solution. This water-soluble polymer coating solution was coated on the bilayered compressed core as prepared above using a pan coater until the coating component accounted for 10% of the mass of the bilayered compressed core.

Further, the obtained water-soluble coating bilayered compressed core was coated with a semipermeable membrane composition. As the semipermeable membrane composition, cellulose acetate and polyethylene glycol 4000 (85:15 (W/W%)) were dissolved to 5% in terms of solids content in acetone/water (95:5 (W/W%)) as a solvent, thus obtaining a coating solution. This coating solution was coated on the water-soluble coating bilayered compressed core produced above using a pan coater until the coating component accounted for 10% of the mass of the bilayered compressed core. The coated core was removed from the pan coater, and then subjected to drying treatment at 40°C for 24 hours using a rack dryer.

The thus-coated bilayered compressed core was provided with drug release ports having a diameter of 0.8 mm on the drug-layer-side surface using an automated laser to prepare an osmotic pump formulation.

Table 1A and Table 1B below show the components of the osmotic pump formulation.

**Table 1A**

| | Formulation (mg/unit) | Example 1-1 | Comparative Example 1-2 |
|---|---|---|---|
| | Core tablet components | | |
| Drug layer | Fumaric acid salt of compound (I) | 38.03 | - |
| | Free base of compound (I) | - | 30.00 |
| | Low-viscosity polyethylene oxide | 60.00 | 60.00 |
| | D-Mannitol | 40.97 | 49.00 |
| | Crospovidone | 20.00 | 20.00 |
| | Magnesium stearate | 1.00 | 1.00 |
| Push layer | High-viscosity polyethylene oxide | 45.40 | 45.40 |
| | D-mannitol | 24.00 | 24.00 |
| | Iron oxide | 0.20 | 0.20 |
| | Magnesium stearate | 0.40 | 0.40 |
| | Subtotal | 230.00 | 230.00 |
| | Coating components | | |
| Water-soluble polymer | HPMC TC-5R | 16.10 | 16.10 |
| | Kollidon K30 | 6.90 | 6.90 |
| Semi-permeable | Cellulose acetate | 19.55 | 19.55 |
| membrane components | PEG 4000 | 3.45 | 3.45 |
| | Subtotal | 46.00 | 46.00 |
| | Total | 276.00 | 276.00 |

**Table 1B**

| | Formulation (mg/unit) | Example 1-3 | Comparative Example 1-4 |
|---|---|---|---|
| | Core tablet components | | |
| Drug layer | Fumaric acid salt of compound (I) | 39.27 | 39.27 |
| | Low-viscosity polyethylene oxide | 99.73 | 119.73 |
| | Hypromellose TC-5R | 20.00 | - |
| | Magnesium stearate | 1.00 | 1.00 |
| Push layer | High-viscosity polyethylene oxide | 45.40 | 45.40 |
| | Sodium hydrogen carbonate | 24.00 | 24.00 |
| | Iron oxide (pigment) | 0.20 | 0.20 |
| | Magnesium stearate | 0.40 | 0.40 |
| | Subtotal | 230.00 | 230.00 |
| | Coating components | | |
| Water-soluble polymer | HPMC TC-5R | 16.10 | 16.10 |
| | Kollidon K30 | 6.90 | 6.90 |
| Semi-permeable membrane components | Cellulose acetate | 31.05 | 31.05 |
| | PEG 4000 | 3.45 | 3.45 |
| | Subtotal | 46.00 | 46.00 |
| | Total | 276.00 | 276.00 |

The formulations obtained in the Examples were subjected to the following Test Example 1, and evaluated.

As the low-viscosity polyethylene oxide, POLYOX (trademark) WSR N-80 (average molecular weight: about 200000, viscosity: 55 to 90 mPa·s (5% W/V aqueous solution, 25°C)) was used. As the high-density polyethylene oxide, POLYOX (registered trademark) WSR Coagulant (average molecular weight: 5000000, viscosity: 5500 to 7500 mPa·s (1% W/V aqueous solution, 25°C)) was used.

### Test Example 1: Measurement of Supersaturated Dissolution Concentration Profile

The release rate from the formulations obtained in the Examples was evaluated by measuring the dissolution rate of each salt of compound (I) at intervals of 1 or 2 hours over 18 or 24 hours. The dissolution test was performed according to the 15th Japanese Pharmacopoeia Dissolution Test Method 2 (paddle method). As a test liquid, 900 mL of the second dissolution test liquid (pH of about 7, potassium dihydrogen phosphate, disodium hydrogen phosphate) listed in the Japanese Pharmacopoeia was used, and the test was performed at 37°C and a paddle rotation speed of 50 rpm. Sampling was performed over time, and the compound (I) (free base) in the sampling solution was quantified with a UV detector (absorbance measurement wavelength: 323 nm and 380 nm). The first wavelength (323 nm) was set as the wavelength at which the absorbance of the main drug can be detected to the maximum, and the second wavelength (380 nm) was set as the wavelength at which the absorbance from the main drug is not detected. In the figure, dissolved (µg/mL) indicates the concentration of the eluted (dissolved) compound (I) (free base).

In Test Example 1, the pH of the eluate was about 7. The results of the dissolution test evaluated in Test Example 1 indicate to what level the salt of compound (I) is eluted in the lower part of the gastrointestinal tract after the oral pharmaceutical composition is orally administered and passed through the stomach. In Test Example 1, the following phenomenon was observed: since the solubility of the salt of the compound (I) is higher than the solubility of compound (I), the dissolution concentration of the salt of compound (I) from the formulation temporarily exceeds the solubility of compound (I) to show a supersaturated concentration, and the dissolution concentration then decreases with recrystallization of compound (I). The profile obtained at that time is defined as a supersaturated dissolution profile, and can be evaluated as an indicator of absorbability in the lower part of the gastrointestinal tract.

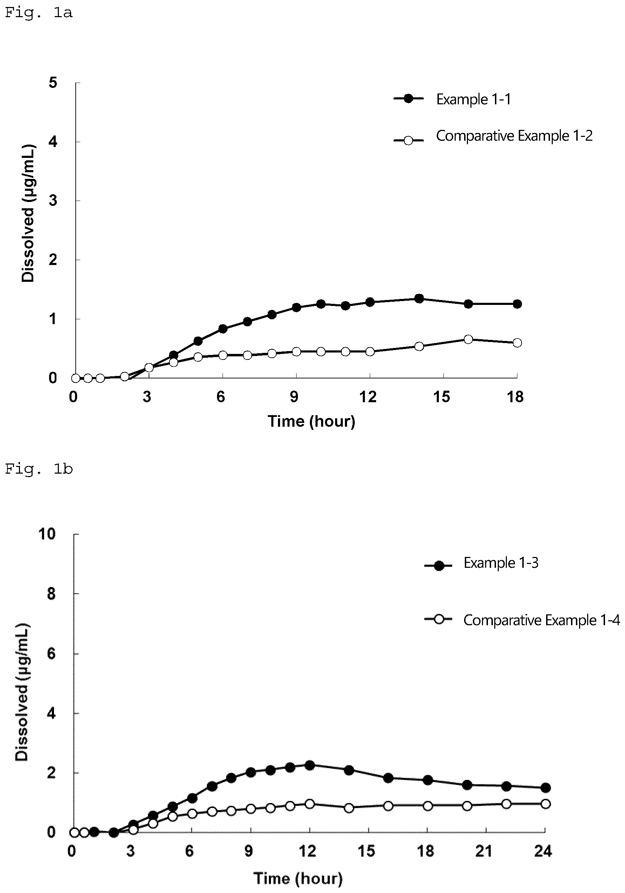

The results of Test Example 1 of the formulations obtained in each Example are shown in Fig. 1(a) or Fig. 1(b). The formulations of the Examples (formulations containing a fumaric acid salt of compound (I)) showed an excellent supersaturated dissolution profile, as compared with the formulations of the Comparative Examples (formulations containing a free base of compound (I)).

### Preparation of Drug Sustained Release Formulation and Evaluation 2

Research was conducted to develop an oral pharmaceutical composition that exhibits an excellent supersaturated dissolution profile. As a result, it was found that when a drug-containing composition is prepared by a combination of a fumaric acid salt of compound (I) with a water-soluble polymer, the composition may possibly exhibit a particularly excellent supersaturated dissolution profile. Accordingly, a fumaric acid salt of compound (I) was combined with various water-soluble polymers; and using these combinations, osmotic pump formulations were prepared according to the compositions shown in Table 2 in the same manner as above. In Table 2, Metolose SM-4 is methyl cellulose, HPC-SL is hydroxypropyl cellulose, PVP Kollidon 25 is polyvinylpyrrolidone, and Kollicoat IR is a polyvinyl alcohol-polyethylene glycol-graft copolymer.

**Table 2**

| | Formulation (mg/unit) | Example 2-1 | Example 2-2 | Example 2-3 | Comparative Example 2-4 | Comparative Example 2-5 | Comparative Example 2-6 |
|---|---|---|---|---|---|---|---|
| | Core tablet component | | | | | | |
| Drug layer | Fumaric acid salt of compound (I) | 39.27 | 39.27 | 39.27 | 39.27 | 39.27 | |
| | Free base of compound (I) | - | - | - | - | - | 30.00 |
| | Low-viscosity polyethylene oxide | 57.73 | 57.73 | 57.73 | 57.73 | 57.73 | 67.00 |
| | Sodium hydrogen fumarate | 40.00 | 40.00 | 40.00 | 40.00 | 40.00 | 40.00 |
| | Hypromellose TC-5R | 20.00 | - | - | - | - | - |
| | Metolose SM-4 | - | 20.00 | - | - | - | |
| | HPC-SL | - | - | 20.00 | - | - | - |
| | PVP Kollidon 25 | - | - | - | 20.00 | - | - |
| | Kollicoat IR | - | - | - | - | 20.00 | 20.00 |
| | Light silicic acid anhydride Aerosol 200 | 2.00 | 2.00 | 2.00 | 2.00 | 2.00 | 2.00 |
| | Magnesium stearate | 1.00 | 1.00 | 1.00 | 1.00 | 1.00 | 1.00 |
| Push layer | High-viscosity polyethylene oxide | 45.40 | 45.40 | 45.40 | 45.40 | 45.40 | 45.40 |
| | Sodium hydrogen carbonate | 24.00 | 24.00 | 24.00 | 24.00 | 24.00 | 24.00 |
| | Iron oxide (pigment) | 0.20 | 0.20 | 0.20 | 0.20 | 0.20 | 0.20 |
| | Magnesium stearate | 0.40 | 0.40 | 0.40 | 0.40 | 0.40 | 0.40 |
| | Subtotal | 230.00 | 230.00 | 230.00 | 230.00 | 230.00 | 230.00 |
| | Coating components | | | | | | |
| Water-soluble polymer | Kollicoat IR | 6.90 | 6.90 | 6.90 | 6.90 | 6.90 | 6.90 |
| Semi-permeable membrane components | Cellulose acetate | 31.05 | 31.05 | 31.05 | 31.05 | 31.05 | 31.05 |
| | PEG 4000 | 3.45 | 3.45 | 3.45 | 3.45 | 3.45 | 3.45 |
| | Subtotal | 41.40 | 41.40 | 41.40 | 41.40 | 41.40 | 41.40 |
| | Total | 271.40 | 271.40 | 271.40 | 271.40 | 271.40 | 271.40 |

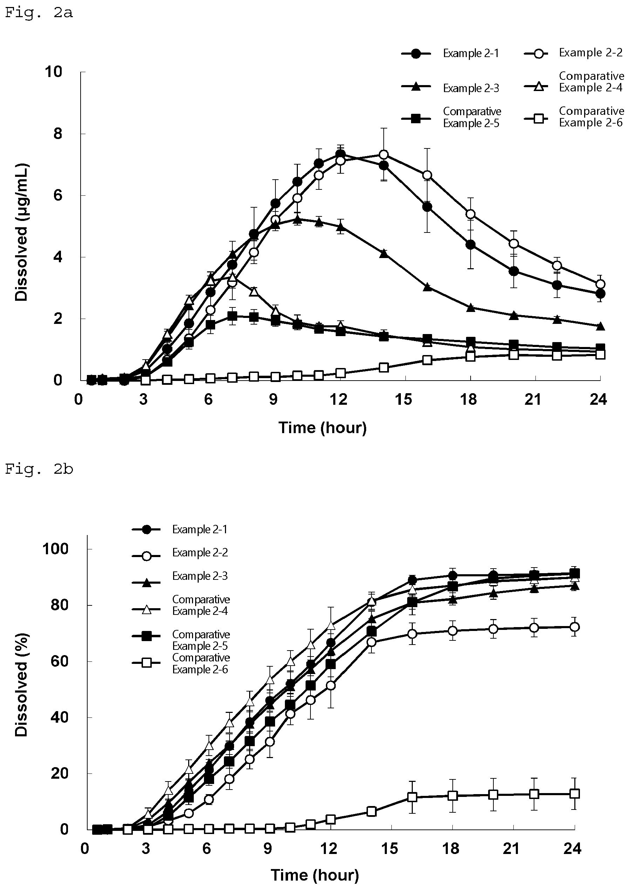

The supersaturated dissolution profile of each formulation obtained in the Examples was also determined in the same manner as in Test Example 1 above. Fig. 2a shows the results. All of the formulations obtained in the Examples showed supersaturated dissolution profiles superior to the supersaturated dissolution profile of the formulation of Example 1-1 (in particular, the peak was higher). Further, in particular, in the examples in which a cellulose-based water-soluble polymer was used, the peak of the supersaturated elution profile was comparatively high, and the use of a cellulose-based water-soluble polymer was thus preferable.

Further, the release rate of these formulations was studied as in Test Example 2 described below.

### Test Example 2: Measurement of Release Rate

The release rate from the formulation of each Example or Comparative Example was evaluated by measuring the dissolution rate of a salt of compound (I) at 30-minute to 2-hour intervals, over 24 hours. The dissolution test was performed according to the 15th Japanese Pharmacopoeia Dissolution Test Method 2 (paddle method). As a test liquid, 900 mL of acetate buffer (pH of about 4.3, acetic acid, sodium acetate) was used, and the test was performed at 37°C and a paddle rotation speed of 50 rpm. Sampling was performed over time, and the compound (I) (free base) in the sampling solution was quantified with a UV detector (absorbance measurement wavelength: 323 nm and 380 nm). The mass ratio (%) of the eluted (dissolved) compound (I) (free base) relative to the total amount (mass) of compound (I) (free base) contained in the formulation taken as 100% was defined as the dissolution rate. The dissolution rate value is the same as the mass ratio (%) of the eluted (dissolved) salt of compound (I) when the total amount (mass) of the salt of compound (I) contained in the formulation is taken as 100%. Accordingly, this value can also be used as the dissolution rate of the salt of compound (I). In the figure showing the results, Dissolved (%) indicates the dissolution rate.

In Test Example 2, the eluate had a pH of about 4.3. The evaluation results of the dissolution test in Test Example 2 is an indicator as to what degree the salt of compound (I) will dissolve in the entire gastrointestinal tract after the oral administration of the oral pharmaceutical composition. The sustained release time of the oral pharmaceutical composition can be evaluated from the obtained profile.

Fig. 2b shows the results. Fig. 2b shows that all of the formulations obtained in the Examples had excellent sustained release.

### Preparation of Drug Sustained Release Formulation and Evaluation 3

Hydrogel sustained release formulations (hydrogel matrix tablets) were produced according to the compositions shown in Table 3. More specifically, the components were mixed and subjected to fluidized bed granulation, and then compressed into hydrogel matrix tablets (uncoated tablets). Hydrogel matrix tablets are a known technique in which the release of a drug is controlled by hydrogel, which is formed by (in the case of enteric-coated tablets, dissolution of the coating film due to a pH increase after gastric excretion, and then) absorption of water in the gastrointestinal tract. In the composition of Example 3-1 shown in Table 3, polyethylene oxide and hypromellose were used as a sustained release base (hydrogel-forming base); and in Comparative Example 3-2 composition, polyethylene oxide was used as a sustained release base (hydrogel-forming base).

**Table 3**

| Formulation (mg/unit) | Example 3-1 | Comparative Example 3-2 |
|---|---|---|
| Fumaric acid salt of compound (I) | 39.27 | 39.27 |
| Polyethylene oxide | 99.73 | 119.73 |
| Sodium hydrogen fumarate | - | - |
| Hypromellose TC-5R | 20.00 | - |
| Magnesium stearate | 1.00 | 1.00 |
| Total | 160.00 | 160.00 |

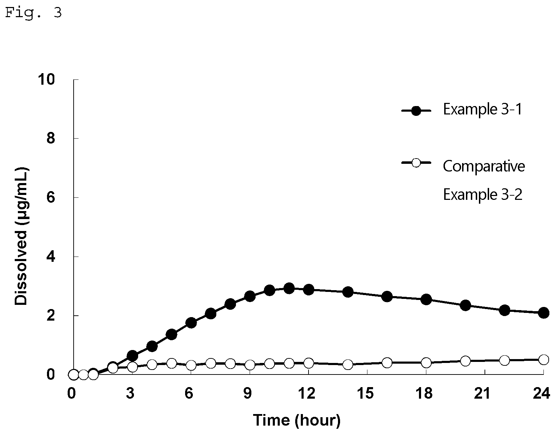

The supersaturated dissolution profile of each of the obtained formulations was also measured in the same manner as in Test Example 1 above. Fig. 3 shows the results. The formulations of the Examples had better supersaturated dissolution profiles than those of the formulations of the Comparative Examples.

### Formulation Example of Osmotic Pump Formulations

Table 4 shows Formulation Examples of osmotic pump formulations containing the oral pharmaceutical composition according to the present disclosure. In Table 4, the amounts of components of the drug layer and the push layer are expressed in parts by mass, whereas the amounts of components of the coating layer are expressed in parts by mass based on 100 parts by mass of the core portion. The "core portion" referred to herein means the portion comprising a combination of the drug layer and the push layer.

**Table 4**

| | | Form. Ex. 1 | Form. Ex. 2 | Form. Ex. 3 | Form. Ex. 4 | Form. Ex. 5 | Form. Ex. 6 | Form. Ex. 7 | Form. Ex. 8 | Form. Ex. 9 | Form. Ex. 10 | Form. Ex. 11 | Form. Ex. 12 | Form. Ex. 13 | Form. Ex. 14 | Form. Ex. 15 | Form. Ex. 16 |
|---|---|---|---|---|---|---|---|---|---|---|---|---|---|---|---|---|---|
| Drug layer | Fumaric acid salt of compound (I) | 7.9 | 9.8 | 10.5 | 11.4 | 12.3 | 13.1 | 13.1 | 13.1 | 15.1 | 17.9 | 17.9 | 17.9 | 23.8 | 24.5 | 24.6 | 24.6 |
| | Polyethylene oxide (MW:200K) | 40.0 | 50.1 | 37.4 | 31.8 | 54.6 | 31.7 | 34.6 | 34.8 | 28.4 | 43.2 | 52.2 | 52.2 | 25.6 | 41.6 | 29.8 | 54.8 |
| | Sodium hydrogen fumarate | 31.7 | 25.0 | 31.7 | 31.9 | 18.8 | 31.2 | 31.7 | 31.7 | 42.5 | 18.2 | 18.2 | 9.1 | 18.8 | 25.0 | 31.3 | 6.3 |
| | Hypromellose | 16.7 | 12.5 | 16.7 | 20.9 | 12.5 | 20.0 | 16.7 | 16.7 | 10.0 | 18.2 | 9.1 | 18.2 | 31.3 | 6.3 | 12.5 | 12.5 |
| | Silicon dioxide | 2.0 | 0.6 | 2.0 | 2.0 | 1.3 | 2.0 | 2.0 | 2.0 | 2.0 | 0.6 | 0.6 | 0.6 | - | 0.6 | 1.3 | 1.3 |
| | Magnesium stearate | 1.8 | 2.0 | 1.8 | 2.0 | 0.6 | 2.0 | 2.0 | 1.8 | 2.0 | 2.0 | 2.0 | 2.0 | 0.6 | 2.0 | 0.6 | 0.6 |
| | Total of the drug layer | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 |
| Push layer | Polyethylene Oxide (MW:5000K) | 64.3 | 64.4 | 64.3 | 64.3 | 64.9 | 64.3 | 64.3 | 64.3 | 64.3 | 64.4 | 64.4 | 64.4 | 64.9 | 64.4 | 64.9 | 64.9 |
| | Sodium hydrogen carbonate | 34.3 | 34.3 | 34.3 | 34.3 | 34.3 | 34.3 | 34.3 | 34.3 | 34.3 | 34.3 | 34.3 | 34.3 | 34.3 | 34.3 | 34.3 | 34.3 |
| | Iron sesquioxide | 0.4 | 0.3 | 0.4 | 0.4 | 0.3 | 0.4 | 0.4 | 0.4 | 0.4 | 0.3 | 0.3 | 0.3 | 0.3 | 0.3 | 0.3 | 0.3 |
| | Magnesium stearate | 1.0 | 1.0 | 1.0 | 1.0 | 0.6 | 1.0 | 1.0 | 1.0 | 1.0 | 1.0 | 1.0 | 1.0 | 0.6 | 1.0 | 0.6 | 0.6 |
| | Total of the push layer | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 |
| Water-soluble polymer (1st coating) | PVA-PEG copolymer/core portion | 3.0 | 3.0 | 3.0 | 5.0 | 2.4 | 5.0 | 3.0 | 3.0 | 5.0 | 5.0 | 5.0 | 5.0 | - | 5.0 | 3.0 | 3.0 |
| | Hypromellose/core portion | - | 7.0 | - | - | 5.6 | - | - | - | - | - | - | - | - | - | 7.0 | 7.0 |
| Semipermeable membrane component (2nd coating) | Cellulose acetate/core portion | 6.3 | 13.5 | 6.3 | 9.0 | 10.8 | 9.0 | 4.8 | 6.3 | 7.2 | 9.0 | 9.0 | 9.0 | 18.0 | 9.0 | 13.5 | 13.5 |
| | PEG4000/core portion | 0.7 | 1.5 | 0.7 | 1.0 | 1.2 | 1.0 | 0.3 | 0.7 | 0.8 | 1.0 | 1.0 | 1.0 | 2.0 | 1.0 | 1.5 | 1.5 |

| | | | | | | | | | | | | | | | | | |
|---|---|---|---|---|---|---|---|---|---|---|---|---|---|---|---|---|---|
| Note: Form. Ex. stands for Formulation Example | | | | | | | | | | | | | | | | | |

### Evaluation of Blood Concentration of Compound (I) After Oral Administration to Humans

The blood concentration of compound (I) in a steady state after oral administration of the oral pharmaceutical composition according to the present disclosure to a human is evaluated based on the following single-dose administration protocol and multiple-dose administration protocol. The formulation (test formulation) used in this evaluation is an osmotic pump formulation prepared according to the present disclosure, which contains a fumaric acid salt of compound (I) as an active ingredient. The doses and contents of the tablets shown in the table below are in terms of the free base, i.e., the weight of compound (I). In the single-dose administration protocol, 24 mg of the test formulation (one 24 mg tablet) is administered once on an empty stomach. Then, after a washout period, 48 mg of the test formulation (two 24 mg tablets) is administered once on an empty stomach. In the multiple-dose administration protocol, the test formulation is repeatedly administrated on an empty stomach by any one of the following administration methods 1 to 5. In each protocol, the PK parameter of compound (I) is analyzed. The observation shows that the formulation maintains a desirable steady-state blood concentration of compound (I) as a drug for a once-a-week administration (for example, 15 ng/mL to 400 ng/mL).

**Table 5**

| Date of administration | First day | | | 8th day, 15th day, 22th day, and 29th day | | |
|---|---|---|---|---|---|---|
| Administration method | Dose | Tablet | Number of tablets | Dose | Tablet | Number of tablets |
| 1 | 24 mg | 24 mg | 1 tablet | 48 mg | 24 mg | 2 tablets |
| 2 | 18 mg | 18 mg | 1 tablet | 36 mg | 18 mg | 2 tablets |
| 3 | 24 mg | 24 mg | 1 tablet | 42 mg | 18 mg | 1 tablet each |
| | | | | | 24 mg | |
| 4 | 30 mg | 30 mg | 1 tablet | 54 mg | 24 mg | 1 tablet each |
| | | | | | 30 mg | |
| 5 | 30 mg | 30 mg | 1 tablet | 60 mg | 30 mg | 2 tablets |

## Claims

1. A controlled release oral solid pharmaceutical composition comprising a fumaric acid salt of 7-[4-(4-benzo [b]thiophen-4-yl-piperazin-1-yl)butoxy]-1H-quinolin-2-one as an active ingredient, and further comprising a cellulose-based water-soluble polymer.

2. The composition according to claim 1, wherein the cellulose-based water-soluble polymer is at least one member selected from the group consisting of hydroxypropyl methylcellulose, hydroxypropyl cellulose, and methyl cellulose.

3. The composition according to claim 1 or 2, which is an osmotic pump composition.

4. The composition according to claim 1 or 2, which is a hydrogel sustained release formulation.

5. The composition according to claim 4, comprising an enteric coating.

6. The composition according to any one of claims 1 to 5, wherein the fumaric acid salt of 7-[4-(4-benzo[b]thiophen-4-yl-piperazin-1-yl)butoxy]-1H-quinolin-2-one is present in an amount of 5 mg to 60 mg in terms of the weight of the free base.

7. The composition according to any one of claims 1 to 6, wherein the blood concentration of 7-[4-(4-benzo[b]thiophen-4-ylpiperazin-1-yl)butoxy]-1H-quinolin-2-one in a steady state when orally administered to a human is maintained in the range of 15 ng/mL to 400 ng/mL for 1 week.

8. The composition according to any one of claims 1 to 7, which is for use in administering the fumaric acid salt of 7-[4-(4-benzo[b]thiophen-4-yl-piperazin-1-yl)butoxy]-1H-quinolin-2-one once a week at a dose of 5 mg to 60 mg in terms of the weight of the free base.

9. The composition according to any one of claims 1 to 8, which is for use in preventing or treating a central nervous system (CNS) disease.

10. The composition according to claim 9, wherein the composition is for preventing or treating a CNS disease selected from the group consisting of schizophrenia; treatment-resistant, refractory, or chronic schizophrenia; emotional disturbance; psychotic disorder; mood disorder; bipolar disorder; depression; endogenous depression; major depression; melancholic and treatment-resistant depression; dysthymic disorder; cyclothymic disorder; anxiety disorder; somatoform disorder; factitious disorder; dissociative disorder; sexual disorder; eating disorder; sleep disorder; adjustment disorder; substance-related disorder; anhedonia, delirium; cognitive impairment; cognitive impairment associated with neurodegenerative disease; cognitive impairment caused by neurodegenerative disease; cognitive impairment of schizophrenia; cognitive impairment caused by treatment-resistant, refractory, or chronic schizophrenia; vomiting; motion sickness; obesity; migraine; pain; mental retardation; autism disorder; Tourette's disorder; tic disorder; attention deficit hyperactivity disorder; conduct disorder; Down syndrome; impulsive symptoms associated with dementia; and borderline personality disorder.
